# FASCICULE DE BREVET EUROPEEN

(11) **EP 1 771 181 B1**
(45) Date de publication et mention de la délivrance du brevet: **10.09.2008**
(21) Numéro de dépôt: 05793737.7
(22) Date de dépôt: 29.07.2005
(51) Int. Cl.: A61K 31/675, A61K 31/555, A61K 33/24, C07F 9/50, A61P 43/00, A61P 35/00, A61P 19/02, A61P 17/06, A61P 27/02, A61P 31/18

(54) **DÉRIVÉS PHOSPHOLES COMPLEXÉS À DES MÉTAUX ET LEURS UTILISATIONS PHARMACEUTIQUES**
METALLKOMPLEXIERTE PHOSPHOLDERIVATE UND PHARMAZEUTISCHE VERWENDUNGEN DAFÜR
PHOSPHOLE DERIVATIVES COMPLEXED WITH METALS, AND PHARMACEUTICAL USES THEREOF

(30) Priorité: 30.07.2004 FR 0408427
(43) Date de publication de la demande: 11.04.2007
(73) Titulaire: CENTRE NATIONAL DE LA RECHERCHE SCIENTIFIQUE (CNRS), 75794 Paris Cedex 16 (FR)
(72) Inventeur: BECKER-BRANDENBURG, Katja, D-35582 Wetzlar-Dutenhofen (DE); DAVIOUD-CHARVET, Elisabeth, F-59840 Perenchies (FR); DEBORDE, Valérie, F-35750 Iffendic (FR); REAU, Régis, 35520 La Chapelle des Fougeretz (FR); SCHIRMER, R., Heiner, 69126 Heidelberg (DE); HEROLD-MENDE, Christel, 69245 Bammental (DE)
(74) Mandataire: Grosset-Fournier, Chantal Catherine
(86) Numéro de dépôt international: PCT/FR2005/002004
(87) Numéro de publication internationale: WO 2006/024770

(56) Documents cités:
- WO-A-01/77121
- DEPONTE M ET AL: "MECHANISTIC STUDIES ON A NOVEL, HIGHLY POTENT GOLD-PHOSPHOLE INHIBITOR OF HUMAN GLUTATHIONE REDUCTASE" JOURNAL OF BIOLOGICAL CHEMISTRY, AMERICAN SOCIETY OF BIOLOCHEMICAL BIOLOGISTS, BIRMINGHAM,, US, vol. 280, no. 21, 27 mai 2005 (2005-05-27), pages 20628-20637, XP008059661 ISSN: 0021-9258
- HAY C ET AL: "A bridging ligand featuring terminal 2-pyridylphosphole moieties: synthesis, optical properties and Ru(II)-complex" JOURNAL OF ORGANOMETALLIC CHEMISTRY, ELSEVIER-SEQUOIA S.A. LAUSANNE, CH, vol. 643-644, 1 février 2002 (2002-02-01), pages 494-497, XP004339612 ISSN: 0022-328X
- SAUTTHIER M ET AL: "A rare phosphane coordination mode: a symmetrically mu2-bridging phosphole in a dinuclear palladium(I) complex" ANGEWANDTE CHEMIE. INTERNATIONAL EDITION, VERLAG CHEMIE. WEINHEIM, DE, vol. 40, no. 1, 5 janvier 2001 (2001-01-05), pages 228-231, XP002223439 ISSN: 0570-0833
- LECA, FRANCOIS ET AL: "Stereospecific isomerization of P-heterocycles triggered by coordination: synthesis of the first P,N-chelates featuring a 2- phospholene moiety" CHEMICAL COMMUNICATIONS (CAMBRIDGE, UNITED KINGDOM) , (14), 1774-1775 CODEN: CHCOFS; ISSN: 1359-7345, 2003, XP008044152
- SAUTHIER M ET AL: "PALLADIUM COMPLEXES OF A NOVEL FAMILY OF P,N-CHELATES, THE 2-(2-PYRIDYL)PHOSPHOLES: SYNTHESIS, STRUCTURAL CHARACTERIZATION AND CATALYTIC ACTIVITY FOR OLEFIN/CO COPOLYMERIZATION" ORGANOMETALLICS, ACS, WASHINGTON, DC, US, vol. 21, no. 8, 15 avril 2002 (2002-04-15), pages 1591-1602, XP001103869 ISSN: 0276-7333 cité dans la demande

## Description

La présente invention a pour objet des compositions pharmaceutiques comprenant des dérivés phospholes complexés à des métaux, et leurs utilisations pour la préparation de médicaments destinés à la prévention ou au traitement de pathologies liées à une activité excessive de la glutathion réductase et/ou de la thiorédoxine réductase.

Les thiorédoxine réductases (TrxR) comme les glutathion réductases (GR) sont des flavoenzymes dimériques NADPH-dépendantes possédant dans leur site catalytique un centre redox actif. La TrxR réduit la thiorédoxine oxydée (Trx), une protéine disulfure de 12 kDa, en thiorédoxine dithiol ; la GR réduit le glutathion disulfure en glutathion réduit (GSH) (Williams, 1992; Holmgren, 2000). La Trx et le GSH sous forme réduits jouent un rôle essentiel dans la régulation cellulaire redox, la défense antioxydante, la régulation enzymatique, l'apoptose et la prolifération cellulaire associée aux tumeurs. Les systèmes basés sur le glutathion et la thiorédoxine fournissent les équivalents réducteurs pour un grand nombre de processus intra- et extra-cellulaires incluant la réduction des acides ribonucléiques, la réduction de composés de faible poids moléculaire comme le lipoamide, l'acide lipoïque, le peroxyde d'hydrogène, les hydroperoxydes lipidiques, le S-nitrosoglutathion, l'ascorbate, l'alloxane, l'ebselen (diselenide), le méthylséléninate, l'ubiquinone et les protéines comme celles se liant au calcium (protéines 1 et 2), la NK-lysine, la protéine disulfure isomérase, et les glutathion peroxydases (Arnér & Holmgren, 2000; Holmgren, 2000; Gromer & Gross, 2002; May *et al*., 2002; Zhong & Holmgren, 2002; Zhao *et al*., 2002; Zhao & Holmgren, 2002; Xia *et al*., 2003). De plus, les Trx et/ou le GSH modulent l'activité des facteurs de transcription comme NF-Y, Pax-8, TTF-1, NF-κB, et servent de facteur d'activation du récepteur glucocorticoïde, jouent un rôle dans la biosynthèse des protéines, ont un impact sur la structure des protéines d'export, régulent l'activité des enzymes, servent d'antioxydants et peuvent agir de façon extracellulaire comme facteur de croissance autocrine (Holmgren, 2000).

D'un point de vue mécanisme catalytique, les électrons sont transférés du NADPH à la flavine adénine dinucléotide (FAD), le groupe prosthétique de ces enzymes, et de là au pont disulfure du site actif (Cys58 et Cys63 dans la GR humaine). Dans les GRs, les électrons sont ensuite transférés du dithiol, généré au cours de la catalyse, au substrat glutathion disulfure. Cependant, dans les TrxRs de mammifères, un centre redox additionnel, représenté par une paire cystéine-selenocystéine localisée dans la partie C-terminale transfère les équivalents réducteurs du premier site actif dans la partie N-terminale au deuxième site actif dans la partie C-terminale. La flexibilité de cette chaîne polypeptidique C-terminale permet l'exposition des équivalents réducteurs à la surface de la protéine, permettant la réduction de la Trx, mais aussi d'un large spectre de différents substrats (Gladyshev *et al*., 1996; Arscott *et al*., 1997; Gromer *et al*., 1997 & 1998; Tamura & Stadtman, 1996; Zhong *et al*., 1998 & 2000). Différentes approches ont démontré que la sélénocysteine est localisée sur un bras accessible extrêmement flexible de la protéine et est essentielle pour le mécanisme catalytique. La première structure tridimensionnelle TrxR de mammifères - la seule décrite jusqu'à maintenant - a été résolue par Sandalova et al., 2001. La structure globale du mutant TrxR de rat étudiée SeCys498Cys est similaire à la GR au niveau des domaines liant le FAD et le NADPH. La structure 3D de la GR humaine est bien connue.

La Trx est secrétée par des cellules normales et néoplasiques. Elle agit sous forme réduite comme un facteur de croissance autocrine (Powis *et al*., 1998). De nombreuses cellules tumorales sont connues pour exprimer des taux accrus en Trx, en glutathion, et des deux réductases TrxR et GR (Kahlos *et al*., 2001; Soini *et al*., 2001). L'implication du système thiorédoxine/TrxR en oncogénèse et en tumorogénèse et son ciblage potentiel en chimiothérapie anticancéreuse ont été récemment confirmés par Lincoln *et al*., 2003. La TrxR mitochondriale a été démontrée pour participer à la régulation de la prolifération cellulaire (Kim *et al*., 2003) et la Trx-1 règle l'expression des gènes spécifiques du cancer du sein (Husbeck & Powis, 2002). La surexpression de la Trx-1 résulte en un accroissement de la production du facteur de croissance vasculaire endothélial, et de l'angiogénèse au niveau de la tumeur (Welsh *et al*., 2002). De plus, puisque la TrxR humaine est inhibée par une variété de principes actifs anticancéreux (comme le cis-Platine, la doxorubicine, la nitrosourée BCNU...), l'enzyme est généralement reconnue pour promouvoir la viabilité et la multiplication cellulaires. Par ailleurs, les GRs et TrxRs jouent un rôle crucial dans les mécanismes de résistance aux médicaments.

Le large spectre fonctionnel des TrxRs et GRs de mammifères, leur implication dans une variété de processus cellulaires et donc leur importance potentielle dans une multitude de maladies rendent extrêmement intéressantes ces deux flavoenzymes comme cibles-candidats pour le développement de médicaments contre les tumeurs, les parasites, et les maladies inflammatoires rhumatoïdes (Sarma *et al*., 2003). Une inactivation spécifique de ces disulfure réductases est en réalité une approche tentante pour, simultanément, diminuer la synthèse d'ADN, la défense antioxydante, la stimulation de croissance autocrine, et la croissance des cellules tumorales. Un nombre de médicaments utilisés en clinique et expérimentalement ont récemment été démontrés pour inhiber les disulfure réductases de façon efficace. Un exemple majeur est l'inhibition des GR et TrxR par l'agent cytostatique BCNU (carmustine). De façon plus intéressante, le BCNU est un des rares agents cytostatiques employés avec succès dans le traitement des glioblastomes. La GR est inhibée par les dérivés isoalloxazines, les inhibiteurs de dimérisation, les dérivés naphtoquinones (Davioud-Charvet *et al*., 2001), et une variété de composés libérant de l'oxyde nitrique (Becker *et al*., 1998 & 2000; Savvides *et al*., 2002). Différents composés ont été décrits pour inhiber la TrxR humaine et pour exercer des effets antitumoraux; ces composés incluent les complexes d'or (I) comme l'aurothioglucose et l'auranofine (Gromer *et al*., 1998; Smith *et al*., 1999), les dérivés trivalents arsenicaux comme le methyl-As(III) (Lin *et al*., 2001), les composés organotellurium solubles dans l'eau, et aussi les naphtoquinones (Wipf *et al*., 2001; Irmler *et al*., 2002 ; Engman *et al*., 2003), et les complexes du cis-diamminedichloroplatine (II) (Arnér *et al*., 2001). De plus, les complexes (2,2':6',2'-terpyridine) platine (II) ont été décrits pour cibler quasi-stoechiométriquement la TrxR humaine, qui est inhibée efficacement *in vitro*. *In vivo*, la prolifération de différentes lignées cellulaires de glioblastomes est fortement inhibée par ces composés (Becker *et al*., 2001; Ross *et al*., 2000).

Les bases de Mannich représentent aussi des inhibiteurs de TrxR d'intérêt (Davioud-Charvet *et al*., 2003) puisque ces composés ont été décrits et développés comme des agents antitumoraux potentiels dans le passé pendant près de 15 ans (Dimmock *et al*., 1998). En considérant l'ensemble de ces données, les inhibiteurs de GRs et de TrxRs sont d'un grand intérêt comme candidat-médicaments cytostatiques et antirhumatismaux et sont d'excellents outils pour étudier la fonction des réseaux cellulaires régulés de façon redox.

La présente invention résulte de la mise en évidence du fait que des dérivés phospholes contenant de l'or et du platine, et dont la synthèse a déjà été décrite (synthèse des phospholes : Hay et al., 2001 ; Sauthier et al., 2002 ; synthèse des complexes de platine : Fave C., 2003 ; synthèse des complexes d'or : Nelsen L., 2002), ou qui n'avaient jamais été synthétisés auparavant, sont des inhibiteurs puissants de la GR et de la TrxR humaines (voir Tableau 1 ci-après), inhibent la croissance de lignée cellulaires tumorales (voir Tableau 2 ci-après) et sont actifs *in vivo* contre des tumeurs chez le rat. Par ailleurs, les Inventeurs ont montré que les dérivés phosholes ci-dessus, non complexés à des métaux, présentaient également une activité d'inhibition de la croissance de lignées cellulaires tumorales (voir Tableau 2 ci-après).

Ainsi la présente invention a essentiellement pour but de fournir de nouvelles compositions pharmaceutiques pour la prévention ou le traitement de pathologies notamment liées à une activité excessive de la glutathion réductase et/ou de la thiorédoxine réductase.

La présente invention a pour objet une composition pharmaceutique, caractérisée en ce qu'elle comprend à titre de composé actif au moins un composé de formule générale (A) suivante : 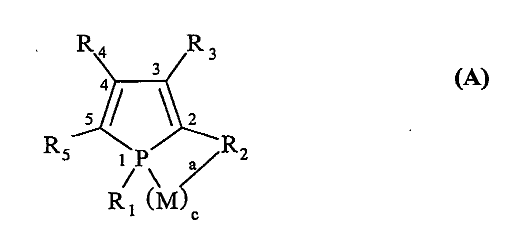 dans laquelle :
- a représente une liaison simple ou aucune liaison,
- c représente 0 lorsque M est absent ou 1 lorsque M est présent,
- R₁ représente un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, tel que le groupement CF₃, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone ;
- le cas échéant (c = 1) M représente un atome métallique, portant éventuellement de 1 à 2 substituants, R₆ et R₇, identiques ou différents, lesquels substituants sont choisis parmi
   - un atome d'halogène,
   - un groupement thio-osidique de 3 à 60 atomes de carbone; éventuellement substitué par un ou plusieurs groupements, identiques ou différents, choisis parmi les groupements protecteurs de groupements hydroxyles et amines, tel qu'un groupement alcanoyle de 2 à 6 atomes de carbone, ou un groupement arylcarbonyle de 6 à 14 atomes de carbone,
   - un groupement peptidique comprenant de 1 à 5 acides aminés, l'un au moins des acides aminés comprenant un groupement thio, tel que la S-cystéine ou le S-glutathion, ou
   - un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, tel qu'un groupe thiophényl, 2-thiopyridyle ou 4-thiopyridyle, éventuellement substitués par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone ;
   en association avec un véhicule pharmaceutiquement acceptable.

Dans un mode de réalisation particulier de l'invention, dans le composé de formule (A) ci-dessus, l'atome métallique M est différent du palladium (Pd), notamment lorsque R₆ et/ou R₇ représentent un atome d'halogène. En effet, la réactivité importante du palladium peut être responsable d'effets indésirables chez le patient auquel est administré un composé de formule (A).

On désigne par « groupement thio-osidique » un goupement comprenant au moins un ose, ledit ose comprenant au moins un groupement thio, en particulier en remplacement d'au moins un groupement hydroxyle dudit ose, et eventuellement un ou plusieurs groupements amines. Plus particulièrement, et le cas échéant, le groupement thio remplace le groupement hydroxyle de la fonction hémiacétalique de l'ose sous forme cyclique. Le groupement osidique selon l'invention peut en particulier comprendre de 1 à 10 oses, dont l'un au moins est un thio-ose.

Selon l'invention le groupement thio-osidique peut notamment représenter : le thioglucose, le thiomannose, le thiogalactose, le thioribose, le thioxylose, le thio-allose, le thiotalose, le thiofucose, le thio-N-acétyl-glucosamine, le thio-N-acétyl-galactosamine, le thio-N-acétyl mannosamine, ou le thiolactose.

Selon l'invention, les groupements protecteurs de groupements hydroxyles et amines représentent notamment : un groupement acétyl, un groupement propionate, un groupement butyrate, ou un groupement benzoyle.

La présente invention a également pour objet une composition pharmaceutique telle que définie ci-dessus, caractérisée en ce qu'elle comprend à titre de composé actif au moins un composé de formule générale (I) suivante : 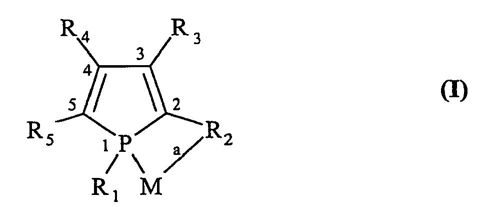 dans laquelle :
- a représente une liaison simple ou aucune liaison,
- R₁ représente un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone ;
- M représente un atome métallique, portant éventuellement de 1 à 2 substituants, R₆ et R₇, identiques ou différents, lesquels substituants sont choisis parmi
   - un atome d'halogène,
   - un groupement thio-osidique de 3 à 60 atomes de carbone, éventuellement substitué par un ou plusieurs groupements, identiques ou différents, choisis parmi les groupements protecteurs de groupements hydroxyles et amines, tel qu'un groupement alcanoyle de 2 à 6 atomes de carbone, ou un groupement arylcarbonyle de 6 à 14 atomes de carbone,
   - un groupement peptidique comprenant de 1 à 5 acides aminés, l'un au moins des acides aminés comprenant un groupement thio, tel que la S-cystéine ou le S-glutathion, ou
   - un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, tel qu'un groupe thiophényl, 2-thiopyridyle ou 4-thiopyridyle, éventuellement substitués par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone ;
   en association avec un véhicule pharmaceutiquement acceptable.

L'invention concerne plus particulièrement une composition pharmaceutique telle que définie ci-dessus, caractérisée en ce qu'elle comprend à titre de composé actif au moins un composé de formule générale (II) suivante : 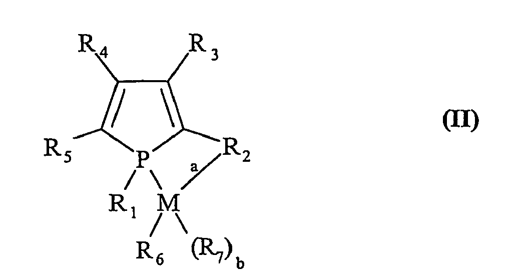 dans laquelle :
- a représente une liaison simple ou aucune liaison,
- b représente 0 ou 1,
- R₁, R₂, R₃, R₄ et R₅ sont tels que définis ci-dessus,
- R₆ et R₇ représentent, indépendamment l'un de l'autre,
   - un atome d'halogène,
   - un groupement thio-osidique de 3 à 60 atomes de carbone, éventuellement substitué par un ou plusieurs groupements, identiques ou différents, choisis parmi les groupements protecteurs de groupements hydroxyles et amines, tel qu'un groupement alcanoyle de 2 à 6 atomes de carbone, ou un groupement arylcarbonyle de 6 à 14 atomes de carbone,
   - un groupement peptidique comprenant de 1 à 5 acides aminés, l'un au moins des acides aminés comprenant un groupement thio, tel que la S-cystéine ou le S-glutathion, ou
   - un groupement thioalkyle linéaire ou ramifié de 1 à 6 atomes de carbone, un groupement thiocycloalkyle saturé ou non saturé saturé de 3 à 7 atomes de carbone ou un groupement thioaryle ou thiohétéroaryle de 6 à 14 atomes de carbone, tel qu'un groupe thiophényl, 2-thiopyridyle ou 4-thiopyridyle, éventuellement substitués par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone ;
- M représente un atome métallique divalent, trivalent ou tétravalent, sous réserve que lorsque M représente un atome métallique divalent alors a ne représente aucune liaison et b vaut 0, lorsque M représente un atome métallique trivalent alors a ne représente aucune liaison et b vaut 1 et que lorsque M représente un atome métallique tétravalent alors a représente une liaison simple et b vaut 1 ;
en association avec un véhicule pharmaceutiquement acceptable.

Dans un mode de réalisation préféré de l'invention, dans les composés de formule (A), (I) ou (II) ci-dessus, M est choisi, le cas échéant, parmi Ag, Cu, Mn, Au et Pt, et notamment parmi Au et Pt

L'invention a plus particulièrement pour objet une composition pharmaceutique comprenant au moins un composé de formule (I) ou (II) telle que définie ci-dessus, caractérisée en ce qu'elle comprend à titre de composé actif au moins un composé de formule générale (III) : 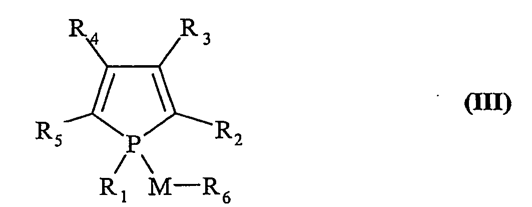 dans laquelle R₁, R₂, R₃, R₄, R₅ et R₆ sont tels que définis ci-dessus, et M représente un atome métallique divalent, tel que Au,
en association avec un véhicule pharmaceutiquement acceptable.

Une composition pharmaceutique comprenant au moins un composé de formule (III) telle que définie ci-dessus particulièrement préférée, est caractérisée en ce qu'elle comprend à titre de composé actif au moins un composé de formule générale (IV) suivante : 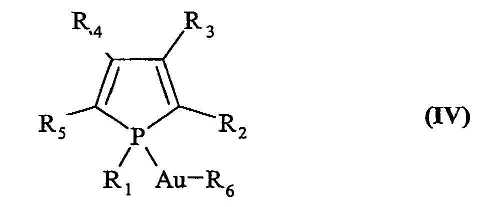 notamment au moins un composé de formule générale (IV') suivante : 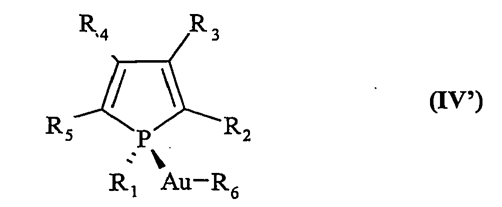 dans lesquelles , R₂, R₃, R₄, R₅ et R₆ sont tels que définis ci-dessus,
en association avec un véhicule pharmaceutiquement acceptable.

L'invention concerne également une composition pharmaceutique comprenant au moins un composé de formule (I) ou (II) telle que définie ci-dessus, caractérisée en ce qu'elle comprend à titre de composé actif au moins un composé de formule générale (V) : 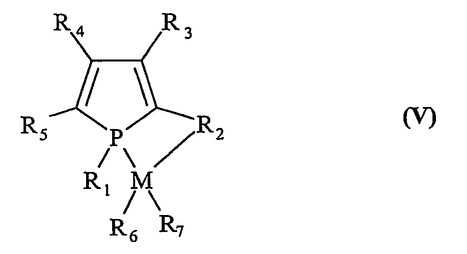 dans laquelle R₁, R₂, R₃, R₄, R₅, R₆ et R₇ sont tels que définis ci-dessus, et M représente un atome métallique tétravalent, tel que Pt,
en association avec un véhicule pharmaceutiquement acceptable.

Une composition pharmaceutique comprenant au moins un composé de formule (V) telle que définie ci-dessus particulièrement préférée, est caractérisée en ce qu'elle comprend à titre de composé actif au moins un composé de formule générale (VI) suivante : 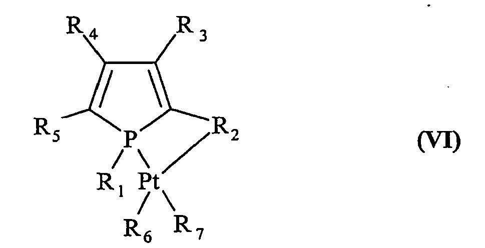 notamment au moins un composé de formule générale (VI') suivante : 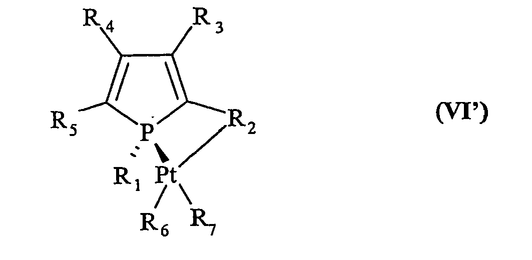 dans lesquelles R₁, R₂, R₃, R₄, R₅, R₆ et R₇ sont tels que définis ci-dessus,
en association avec un véhicule pharmaceutiquement acceptable.

L'invention a plus particulièrement pour objet une composition pharmaceutique comprenant au moins un composé de formule (VI) telle que définie ci-dessus,
caractérisée en ce qu'elle comprend à titre de composé actif au moins un composé de formule générale (VII) suivante : 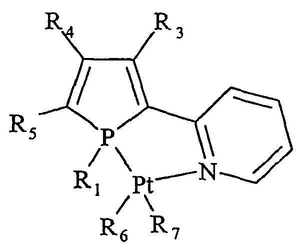 notamment au moins un composé de formule générale (VII') suivante : 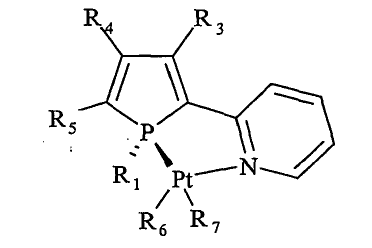 dans lesquelles R₁, R₃, R₄, R₅, R₆ et R₇ sont tels que définis ci-dessus,
en association avec un véhicule pharmaceutiquement acceptable.

L'invention a également pour objet toute composition pharmaceutique telle que définie ci-dessus, caractérisée en ce que :
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle ou pyridyle , substitué ou non, et plus particulièrement un groupement phényle, 2-thiényle ou 2-pyridyle ;
- R₆ et le cas échéant R₇ représentent indépendamment l'un de l'autre un halogène, et plus particulièrement un atome de chlore, un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiolactose, le thiolactose hepta-acétate.

L'invention a plus particulièrement pour objet une composition pharmaceutique telle que définie ci-dessus, caractérisée en ce qu'elle comprend à titre de composé actif au moins un composé de formule suivante : 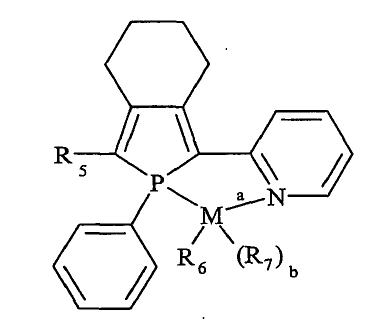 dans laquelle R₅ représente un groupe 2-thiényle ou 2-pyridyle, M représente Au ou Pt, a représente une simple liaison lorsque M représente Pt ou aucune liaison lorsque M représente Au, b représente 1 lorsque M représente Pt et 0 lorsque M représente Au, et R₆ et R₇ sont tels que définis ci-dessus.

L'invention concerne plus particulièrement une composition pharmaceutique telle que définie ci-dessus, caractérisée en ce qu'elle comprend à titre de composé actif au moins un composé de formule suivante : 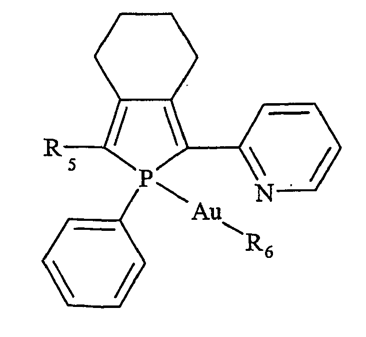 dans laquelle R₅ représente un groupe 2-thiényle ou 2-pyridyle et R₆ est tel que défini ci-dessus.

L'invention a plus particulièrement pour objet toute composition pharmaceutique telle que définie ci-dessus, caractérisée en ce qu'elle comprend à titre de composé actif au moins un composé de formule (1), (2), (3), (4), ou (8) suivante :

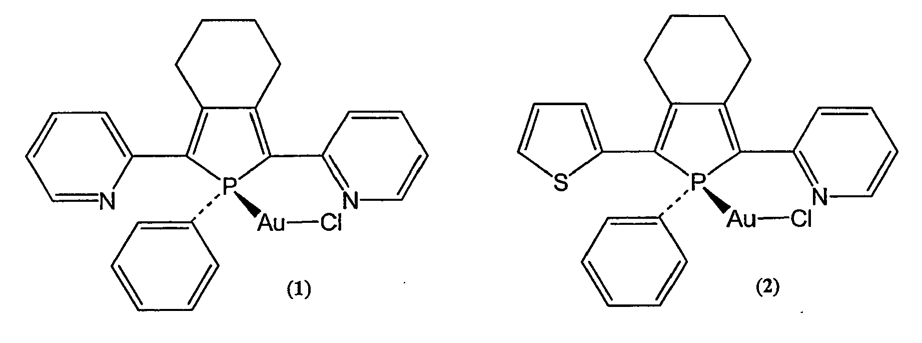

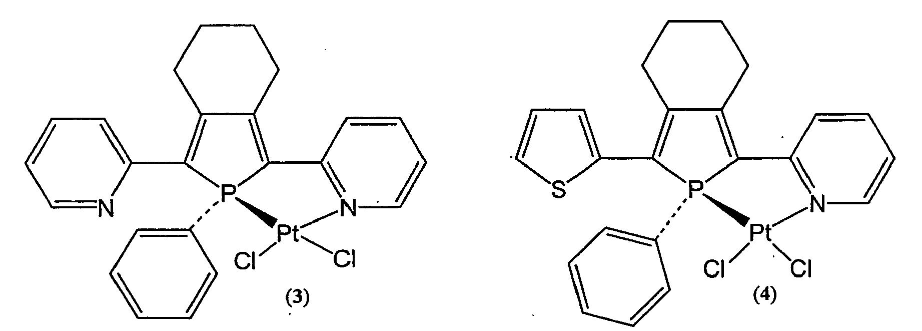

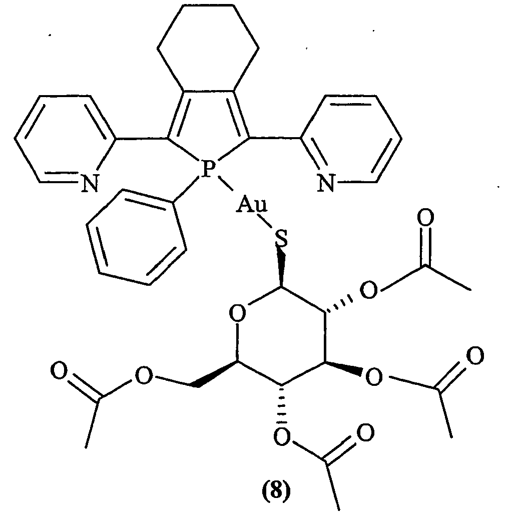

La présente invention concerne également une composition pharmaceutique telle que définie ci-dessus, caractérisée en ce qu'elle comprend à titre de composé actif au moins un composé de formule générale (A) correspondant en particulier à la formule générale (VIII) suivante : 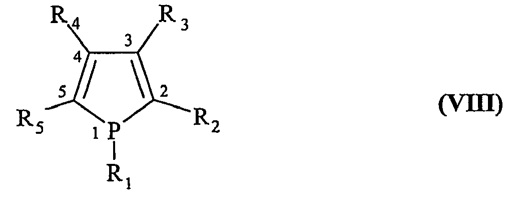 dans laquelle :
- R₁ représente un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone ;
   en association avec un véhicule pharmaceutiquement acceptable.

Selon un mode de réalisation particulier de la composition pharmaceutique comprenant au moins un composé de formule (VIII) telle que définie ci-dessus :
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle , ou pyridyle , substitué ou non, et plus particulièrement un groupement phényle, 2-thiényle ou 2-pyridyle.

L'invention concerne plus particulièrement une composition pharmaceutique comprenant un composé de formule (VIII) telle que définie ci-dessus, caractérisée en ce qu'elle comprend à titre de composé actif au moins un composé de formule suivante : 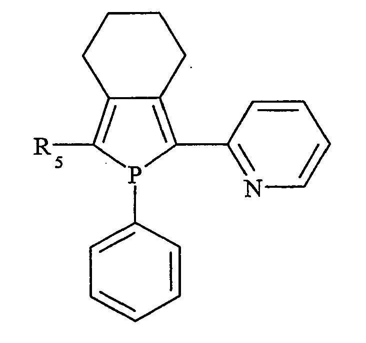 dans laquelle R₅ est tel que défini ci-dessus.

Selon un autre mode de réalisation préféré de la composition comprenant au moins un composé de formule (VIII) telle que définie ci-dessus, ladite composition comprend à titre de composé actif au moins un composé de formule suivante :

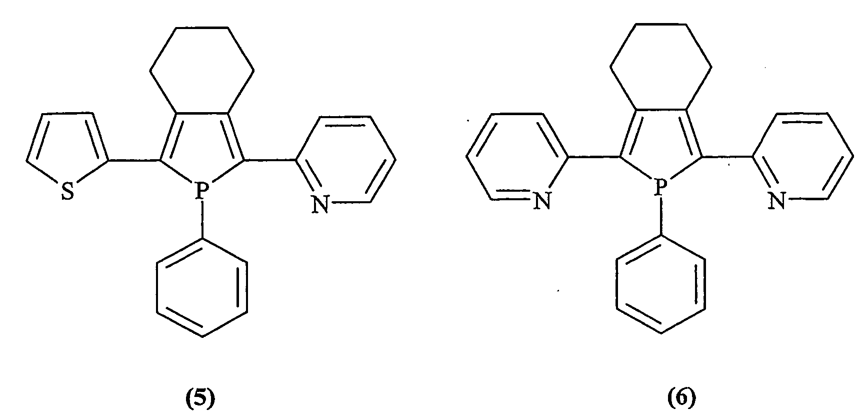

L'invention concerne également toute composition pharmaceutique telle que définie ci-dessus, caractérisée en ce qu'elle comprend à titre de composé actif au moins un composé de formule générale (A), et plus particulièrement de formule (I) à (VIII), tel que défini ci-dessus, en association avec au moins un composé anticancéreux tels que le cisplatin, la mitomycine C, la doxorubicine, l'étoposide, la carmustine.

A ce titre, l'invention a plus particulièrement pour objet les produits comprenant:
* au moins un composé de formule générale (A), et plus particulièrement de formule (I) à (VIII), tel que défini ci-dessus,
* et au moins un composé anticancéreux, tel que défini ci-dessus,
comme produits de combinaison pour une utilisation simultanée, séparée ou étalée dans le temps, en thérapie cancéreuse.

Avantageusement, les produits de combinaison susmentionnés contiennent un produit de formule (A), notamment de formule (I), et un agent anticancéreux dans un rapport d'environ 0,1/1 à environ 2,5/1 et, le cas échéant, un ou plusieurs véhicules pharmaceutiquement acceptables.

De préférence, les compositions pharmaceutiques, ou produits de combinaison susmentionnés, se présentent sous une forme administrable par voie intraveineuse.

Avantageusement encore, la posologie des composés de formule (A), notamment de formule (I), contenus dans les compositions pharmaceutiques ou produits de combinaison susmentionnés, est d'environ 5 à environ 200 mg/m²/j ou par cure.

L'invention concerne également l'utilisation d'au moins un composé de formule générale (I), et plus particulièrement de formule (II) à (VIII), tel que défini ci-dessus, pour la préparation d'un médicament destiné à la prévention ou au traitement de pathologies liées à une activité excessive de la glutathion réductase et/ou de la thiorédoxine réductase.

Une activité excessive de la glutathion réductase et/ou de la thiorédoxine réductase peut être détectée selon les méthodes suivantes. Elles sont basées sur le dosage, soit de la concentration en protéine, ou de l'activité enzymatique, ou de la concentration en produit de la réaction catalysée (glutathion, thiorédoxine), ou de la concentration en ARNm.

L'excès de protéine est difficile à mettre en évidence par Westem-blot, à cause de la teneur faible de ces enzymes dans la cellule. Il existe des anticorps anti-glutathion réductase et des anticorps anti-thiorédoxine réductase.

La méthode la plus fiable consiste à prendre un nombre de cellules exact, à préparer un extrait et à mesurer l'activité enzymatique par spectrophotométrie classique, en mesurant l'oxydation du NADPH au cours de la réduction du glutathion disulfure ou de la thiorédoxine oxydée. La comparaison de l'activité obtenue à celle d'un même nombre de cellules saines, comme contrôle, permet d'évaluer l'excès en activité disulfure réductase.

Une autre méthode consiste aussi à mesurer le potentiel rédox dans une seule cellule par méthode électrochimique. Comme le glutathion est le thiol majoritaire dans la cellule, la mesure du potentiel redox dans le cytosol permet de donner une image très exacte du ratio GSH/GSSG. Pour connaître le ratio thioredoxine/thioredoxine réduite, il faut faire le même traitement en présence de diamide.

Les méthodes de mesures du taux de glutathion sont nombreuses. Cependant, elles ont toutes pour défaut de donner une image peu exacte de la réalité. Quand un extrait cellulaire est préparé, de nombreuses réactions (oxydation à l'air, conditions de lyse des cellules, ...) modifient le ratio initial glutathion réduit/glutathion oxydé. Si bien que les résultats de la littérature sont peu fiables.

Généralement c'est plutôt au niveau des ARNm que l'excès peut être mis en évidence par Northern-blot.

Parmi les pathologies liées à une activité excessive de la glutathion réductase et/ou de la thiorédoxine réductase, susceptibles de pouvoir être traitées dans le cadre de la présente invention, on peut citer notamment :
- les cancers, à savoir toutes tumeurs liquides ou solides, et plus particulièrement les cancers associés à l'infection par le virus d'Epstein-Barr,
- la polyarthrite rhumatoïde,
- le psoriasis ou les rhumatismes psoriasiques,
- le syndrome de Sjögren,
- l'infection au virus HIV et maladies associées comme la carcinome de Kaposi.

La présente invention concerne également l'utilisation d'au moins un composé de formule (VIII) tel que défini ci-dessus, pour la préparation d'un médicament destiné au traitement des cancers.

L'invention a également pour objet l'utilisation d'au moins un composé de formule générale (A), et plus particulièrement de formule (I) à (VIII), tel que défini ci-dessus, pour la préparation d'un médicament destiné à la prévention ou au traitement des phénomènes de résistance aux médicaments anticancéreux tels que le cisplatin, la mitomycine C, la doxorubicine, l'étoposide, la carmustine, et, le cas échéant, aux médicaments antinfectieux tels que le métronidazole, l'isoniazide.

La présente invention concerne également les composés de formule générale (IX) suivante : 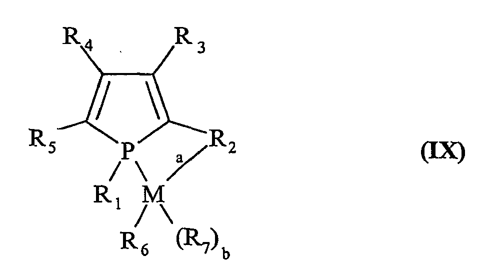 dans laquelle :
- a représente une liaison simple ou aucune liaison,
- b représente 0 ou 1,
- R₁ représente un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
   - alkyle linéaire ou ramifié de 1 à 6 atomes de carbone,
   - cycloalkyle saturé ou non saturé de 3 à 7 atomes de carbone,
   - aryle de 6 à 14 atomes de carbone,
   - alkoxy linéaire ou ramifié de 1 à 6 atomes de carbone,
   - cycloalkoxy saturé ou non saturé de 3 à 7 atomes de carbone,
   - aryloxy de 6 à 14 atomes de carbone,
   - alkylthio linéaire ou ramifié de 1 à 6 atomes de carbone,
   - cycloalkylthio saturé ou non saturé de 3 à 7 atomes de carbone, ou
   - arylthio de 6 à 14 atomes de carbone,
   éventuellement substitué par un ou plusieurs groupements, identiques ou différents, choisis parmi un atome d'halogène, en particulier F, Cl, Br, ou I, notamment F, un groupement alkyle linéaire ou ramifié de 1 à 6 atomes de carbone, éventuellement substitué par un ou plusieurs atomes d'halogène, notamment CF₃, ou un groupement aryle de 6 à 14 atomes de carbone ;
- M représente un atome métallique divalent, trivalent ou tétravalent, sous réserve que lorsque M représente un atome métallique divalent alors a ne représente aucune liaison et b vaut 0, lorsque M représente un atome métallique trivalent alors a ne représente aucune liaison et b vaut 1 et que lorsque M représente un atome métallique tétravalent alors a représente une liaison simple et b vaut 1,
- R₆ représente un groupement thio-osidique de 3 à 60 atomes de carbone, éventuellement substitué par un ou plusieurs groupements, identiques ou différents, choisis parmi les groupements protecteurs de groupements hydroxyles et amines, tel qu'un groupement alcanoyle de 2 à 6 atomes de carbone, ou un groupement arylcarbonyle de 6 à 14 atomes de carbone,
- R₇ représente
   - un atome d'halogène,
   - un groupement thio-osidique de 3 à 60 atomes de carbone, éventuellement substitué par un ou plusieurs groupements, identiques ou différents, choisis parmi les groupements protecteurs de groupements hydroxyles et amines, tel qu'un groupement alcanoyle de 2 à 6 atomes de carbone, ou un groupement arylcarbonyle de 6 à 14 atomes de carbone,
   - un groupement peptidique comprenant de 1 à 5 acides aminés, l'un au moins des acides aminés comprenant un groupement thio, tel que la S-cystéine ou le S-glutathion, ou
   - un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, tel qu'un groupe thiophényl, 2-thiopyridyle ou 4-thiopyridyle, éventuellement substitués par un ou plusieurs groupements, identiques ou différents, choisis parmi un atome d'halogène, en particulier F, Cl, Br, ou I, notamment F, un groupement alkyle de 1 à 6 atomes de carbone, éventuellement substitué par un ou plusieurs atomes d'halogène, notamment CF₃, ou un groupement aryle de 6 à 14 atomes de carbone.

Dans un mode de réalisation particulier, la présente invention concerne un composé de formule générale (IX) définie ci-dessus, correspondant plus particulièrement au composé de formule générale (X): 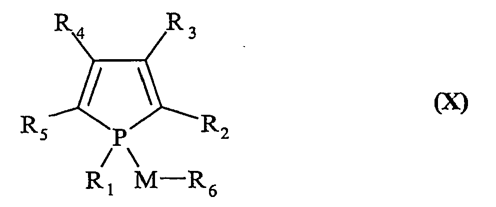 dans laquelle R₁, R₂, R₃, R₄, R₅ et R₆ sont tels que définis ci-dessus, et M représente un atome métallique divalent, tel que Au.

Dans un autre mode de réalisation particulier, l'invention concerne un composé de formule générale (IX) ou (X) définie ci-dessus, correspondant plus particulièrement au composé de formule générale (XI): 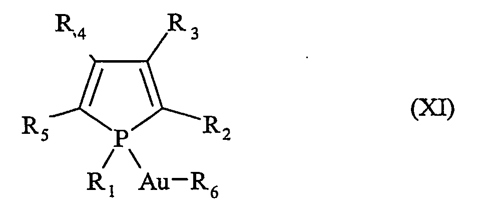 dans laquelle R₂, R₃, R₄, R₅ et R₆ sont tels que définis ci-dessus.

Dans un autre mode de réalisation particulier, l'invention concerne un composé de formule (IX), (X), ou (XI) dans laquelle :
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle ou pyridyle , substitué ou non, et plus particulièrement un groupement phényle, 2-thiényle ou 2-pyridyle ;
- R₆ représente un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiolactose, le thiolactose hepta-acétate ;
- le cas échéant, R₇ représente un halogène, et plus particulièrement un atome de chlore, un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiolactose, le thiolactose hepta-acétate.

L'invention concerne plus particulièrement un composé de formule (IX), (X) ou (XI) tel que définie ci-dessus, de formule (XII) suivante : 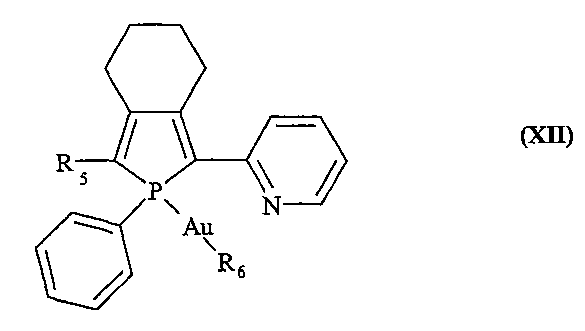 dans laquelle R₅ et R₆ sont tels que définis ci-dessus.

Dans un mode de réalisation préféré, l'invention concerne un composé de formule (IX), (X), (XI) ou (XII) correspondant plus particulièrement au composé de formule (8) suivante :

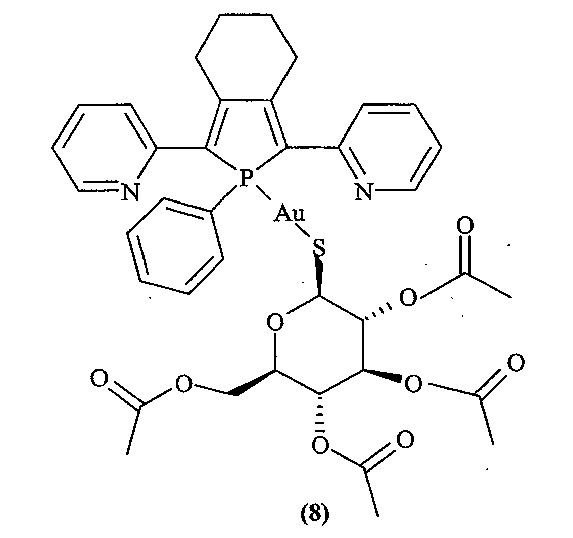

Avantageusement les composés de formule (IX), (X), (XI), (XII), ou (8) sont solubles en solvants aqueux et stables dans le temps. La partie sucre (osidique) protégée au niveau des groupes hydroxyles, par exemple par des groupements alcanoyles, comme des acétates, confère une perméabilité orale alors qu'une partie sucre non protégée (fonctions hydroxyles libres) confère une activité similaire au complexe protégé mais avec une administration par voie injectable.

L'invention sera davantage illustrée à l'aide de la description détaillée qui suit de la synthèse de dérivés phospholes utilisés dans le cadre de la présente invention, et de la démonstration de leurs propriétés d'inhibition de la GR et de la TrxR humaines.

Les 2-pyridylphospholes sont accessibles par diverses méthodes connues de l'homme de métier. On peut citer les références suivantes : (a) C. Hay, D. Le Vilain, V. Deborde, L. Toupet, R. Réau Chem. Commun., 1999, 345-346 (b) Holand, S.; Jeanjean, M.; Mathey, F. Angew. Chem., Int. Ed. 1997, 36, 98.(c) M. Sauthier, F. Leca, L. Toupet, R. Réau Organometallics, 2002, 3 21, 1591.

Plus particulièrement, les 2-pyridylphospholes (5) et (6) suivantes sont décrites dans les publications (a) et (c), respectivement.

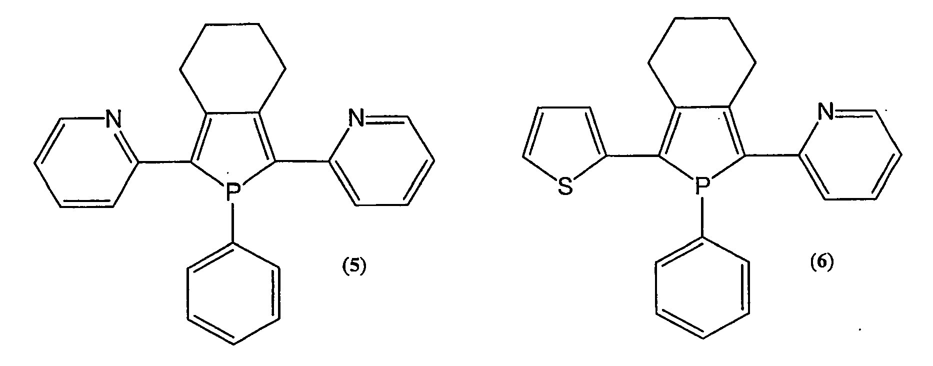

La réaction d'un composé (5) ou (6) avec un complexe de formule LAuX ou HAuCl₄, 3 H₂O pour obtenir le complexe de formule (1) ou (2) correspondant susmentionné peut être menée sous atmosphère inerte (argon, azote) dans un solvant organique usuel à une température comprise entre -90°C et +80°C.

Les composés (1) et (2) sont purifiés par des méthodes classiques de purification (lavages et cristallisation à froid). Ces composés sont décrits dans le rapport de Diplôme d'Etude Approfondi de Chimie Moléculaire de N. Lessen, Université de Rennes 1, juin 2002.

La réaction d'un composé (5) ou (6) avec un complexe de formule L₂PtX₂ pour obtenir le complexe (3) ou (4) correspondant peut être menée sous atmosphère inerte (argon, azote) dans un solvant organique usuel à une température comprise entre -90°C et +80°C. Les composés (3) et (4) sont purifiés par des méthodes classiques de purification (lavages et cristallisation à froid).

Ces composés sont décrits dans le mémoire de Doctorat de l'Université de Rennes 1 de C. Fave, octobre 2003.

Dans les précurseurs cités ci-dessus LAuX et L₂PtX₂, L peut être un ligand de type base de Lewis, et plus particulièrement un ligand soufré tel que le diméthylsulfure ou le tétrahydrothiophène, ou azoté tel que l'acétonitrile ou le benzonitrile. X est un atome d'halogène, tel que défini précédemment. Ces complexes sont commerciaux ou accessibles par les méthodes connues de l'homme de métier.

Le composé (8) est obtenu en déprotonnant la fonction thiol du 1-thio-β-D-glucose-tétraacétate en solvant organique puis en le faisant réagir avec le composé (1).

En tant que solvant organique usuel, on peut utiliser des hydrocarbures aromatiques (benzène, toluène...), des éthers (diethyléther, tétrahydrofurane...), des halogénoalcanes (dichlorométhane, chloroforme...) ainsi que des alcool aliphatiques (méthanol, éthanol...).

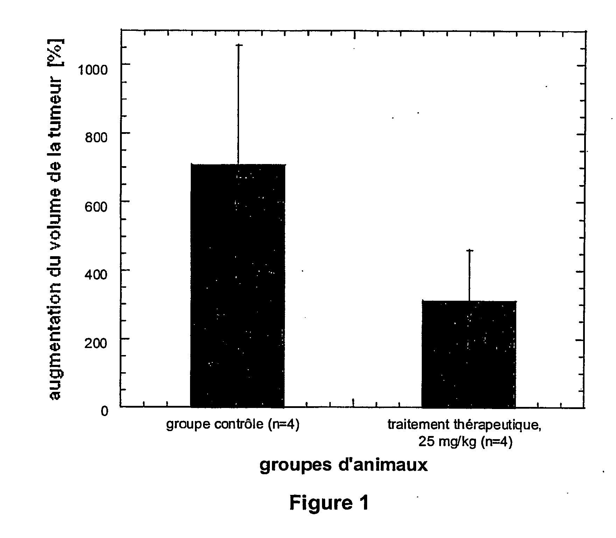

### Description de la Figure 1

La figure 1 représente l'augmentation du volume de tumeurs (axe des ordonnées, en pourcentage) implantées dans le cerveau de 4 rats contrôles (histogramme de gauche) et de 4 rats traités par le complexe (1) (histogramme de droite) à la dose de 25 mg/kg administré par voie intraveineuse trois fois consécutivement pendant le développement de la tumeur.

### Exemples

Les exemples 1 à 5 suivants sont présentés pour illustrer les procédures de synthèse .

### Exemple 1

### Synthèse du complexe (1)

A une solution de CH₂Cl₂ (10 mL) du of 1-phényl-2,5-di(2-pyridyl)phosphole (5) (0.030 g, 0.081 mmol), du AuCl(tétrahydrothiophène) (0.026 g, 0.081 mmol) est ajouté sous forme de poudre à température ambiante. Le mélange est laissé trois heures sous agitation à température ambiante, puis les produits volatils sont évaporés sous vide. Le solide est lavé avec du pentane (4 x 10 mL) et le complexe (1) est obtenu sous forme d'un solide stable à l'air (0,034 g, 70% de rendement).
¹H NMR (300 MHz, CDCl₃): δ 1,85 (m, 4H), 3,06 (m, 2H), 3,24 (m, 2H), 7,09 (dd, *J*(H,H) = 7,2 Hz, J(H,H) = 4,7 Hz, 2H), 7,30 (m, 3H), 7,68 (m, 6H), 8,51 (d, 2H, *J*(H,H) = 4,7 Hz),
¹³C-{¹H} NMR (CDCl₃; 75,46 MHz ): δ 22,7 (s), 30,0 (s), 30,2 (s), 122,8 (s), 124,5 (d, *J*(P,C) = 5,5 Hz), 129,3 (d, *J*(P,C) = 12,4 Hz), 132,2 (s), 134,5 (d, *J*(P,C) = 14,0 Hz) 137,2 (s), 149,4 (s), 152,2 (d, *J*(P,C) = 13,9 Hz), 153,6 (m) ; certains atomes de carbone quaternaires ne sont pas observés,
³¹P-{¹H} NMR (CDCl₃, 121,5 MHz): δ +39,9 (s),
Spectrométrie de masse haute résolution (*m*NBA, FAB): (*mlz*) 601,0859 *[M*+*H*]⁺ ,
UV-Vis, (CH₂Cl₂) λmax (nm), ε (M⁻¹ cm⁻¹) : 271 (7850), 383 (8300), Emission (CH₂Cl₂) λ em (nm) : 495.

### Exemple 2

### Synthèse du complexe (2)

A une solution de CH₂Cl₂ (10 mL) du 1-phényl-2-(2-pyridyl)-5-(2-thiényl)phosphole (6) (0,092 g, 0,25 mmol), du AuCl(tétrahydrothiophène) (0,079 g, 0,25 mmol) est ajouté sous forme de poudre à température ambiante, Le mélange est laissé trois heures sous agitation à température ambiante, puis les produits volatils sont évaporés sous vide, Le solide est lavé avec du pentane (4 x 10 mL) et le complexe (2) est obtenu sous forme d'un solide stable à l'air (0,105 g, 70% de rendement),
¹H NMR (300 MHz, CDCl₃): δ 1,85-2,10 (m, 4H), 2,95-3,35 (m, 4H), 6,99 (dd, *J*(H,H) = 4,5 Hz, 3 *J*(H,H) = 4,0 Hz, 1H), 7,11 (ddd, *J*(H,H) = 2,3 Hz, *J*(H,H) = 4,6 Hz, *J*(H,H) = 6,8 Hz, 1H), 7,44-7,52 (m, 2H), 7,65-7,59 (m, 2H), 7,77 (ddd, J(H,H) = 1,3 Hz, *J*(H,H) = 7,4 Hz, *J*(H,H) = 8,0 Hz, 1H), 8,52 (dd, 2H, *J*(H,H) = 4,6 Hz, *J*(H,H) = 1,6 Hz),
¹³C-{¹H} NMR (CDCl₃; 75,46 MHz ): δ 22,7 (s), 22,8 (s), 29,7 (d, *J*(P,C) = 10,2 Hz), 30,5 (d, *J*(P,C) = 9,9 Hz), 122,4 (s), 123,5 (d, *J*(P,C) = 7,1 Hz), 127,8 (s), 128,1 (s), 128,8 (d, *J*(P,C) = 7,8 Hz), 129,6 (d, *J*(P,C) = 12,5 Hz), 132,5 (d, *J*(P,C) = 3,1 Hz), 134,4 (d, *J*(P,C) = 14,0 Hz) 136,7 (s), 149,7 (s); certains atomes de carbone quaternaires ne sont pas observés,
^{3l}P-{¹H} NMR (CDCl₃, 121,5 MHz): δ +40,2 (s),
Spectrométrie de masse haute résolution (*m*NBA, FAB): (*m*/*z*) 606,0 [*M*+*H*]⁺.

### Exemple 3

### Synthèse du complexe (4)

A une solution de CH₂Cl₂ (10 mL) du 1-phényl-2,5-di(2-pyridyl)phosphole (5) (0,10 g, 0,27 mmol), du (PhCN)₂PtCl₂ (0,128 g, 0,27 mmol) est ajouté sous forme de poudre à température ambiante, Le mélange est laissé une heure sous agitation à température ambiante, puis les produits volatils sont évaporés sous vide. Le solide est lavé avec à l'éther diéthylique (3 x 10 mL) et le complexe (3) est obtenu sous forme d'un solide stable à l'air (86% de rendement),
¹H NMR (300 MHz, CDCl₃): δ 1,70-2,00 (m, 4H), 2,50-2,75 (m, 1H), 2,85-3,22 (m, 3H), 7.08 (dd, *J*(H,H) = 7.3 Hz, 3 *J*(H,H) = 4,9 Hz, 1H), 7.72-8.00 (m, 9H), 8.55 (d, *J*(H,H) = 8.0 Hz, 1H), 8.65 (d, *J*(H,H) = 4.1 Hz, 1H), 9.94 (d, *J*(H,H) = 6.1 Hz, 1H),
¹³C-{¹H} NMR (CDCl₃; 75,46 MHz ): δ 21,2 (s), 22,8 (s), 28,1 (d, *J*(P,C) = 9,4 Hz), 30,6 (d, *J*(P,C) = 10,1 Hz), 123,0 (s), 124,2 (s), 127,9 (s), 129,4 (d, *J*(P,C) = 12,2 Hz), 129,5 (s), 132,9 (d, *J*(P,C) = 2,8 Hz), 134,2 (d, *J*(P,C) = 12,7 Hz), 139,2 (s), 149,8 d, *J*(P,C) = 13,6 Hz), 149,5 (s), 149,8 (d, *J*(P,C) = 12,1 Hz), 152,1 (d, *J*(P,C) = 18,4 Hz), 152,8 (d, *J*(P,C) = 14,0 Hz), 153,1 (s).
³¹P-{¹H} NMR (CDCl₃, 121,5 MHz): δ +35,7 (s, *J*(P,Pt) = 3712 Hz),
Spectrométrie de masse haute résolution (*o*NPOE, FAB): (*m*/*z*) 635,0568 [*M*+*H*]⁺.

### Exemple 4

### Synthèse du complexe (4)

A une solution de CH₂Cl₂ (10 mL) du 1-phényl-2-(2-pyridyl)-5-(2-thiényl)phosphole (6) (0,07 g, 0,20 mmol), du (PhCN)₂PtCl₂ (0,09 g, 0,20 mmol) est ajouté sous forme de poudre à température ambiante. Le mélange est laissé une heure sous agitation à température ambiante, puis les produits volatils sont évaporés sous vide, Le solide est lavé avec à l'éther diéthylique (3 x 10 mL) et le complexe (4) est obtenu sous forme d'un solide stable à l'air (0,11 g, 86% de rendement),
¹H NMR (300 MHz, CDCl₃): δ 1,62-2,00 (m, 4H), 2,60-2,73 (m, 2H), 3,07-2,86 (m, 2H), 7,65-7,59 (m, 2H), 6,26 (d, *J*(H,H) = 4,3 Hz, 1H), 7,10-7,90 (m, 9H), 9,88 (d, 2H, *J*(H,H) = 5,8 Hz),
¹³C-{¹H} NMR (CDCl₃; 75,46 MHz ): δ 21,5 (s), 22,4 (s), 27,9 (d, *J*(P,C) = 9,3 Hz), 29,5 (d, *J*(P,C) = 10,6 Hz), 122,4 (s), 123,4 (d, *J*(P,C) = 9,7 Hz), 123,5 (s), 124,3 (d, *J*(P,C) = 57,3 Hz), 127,7 (s), 128,8 (d, *J*(P,C) = 12,4 Hz), 129,1 (s), 130,5 (d, *J*(P,C) = 60.6 Hz), 132,6 (d, *J*(P,C) = 2,9 Hz), 133,5 (d, *J*(P,C) = 12,9 Hz), 133,9 (d, *J*(P,C) = 4,1 Hz), 135,3 (d, *J*(P,C) = 19,4 Hz), 135,8 (d, *J*(P,C) = 57,5 Hz), 138,9 (s), 148,3 (d, *J*(P,C) = 15,8 Hz), 150,8 (d, *J*(P,C) = 12,0 Hz), 152,1 (s),
³¹P-{¹H} NMR (CDCl₃, 121,5 MHz): δ +37,3 (s, *J*(P,Pt) = 3704 Hz).
Spectrométrie de masse haute résolution (*m*NBA, FAB): (m/z) 602,0375 [*M* -Cl]⁺.

### Exemple 5

### Synthèse du complexe (8)

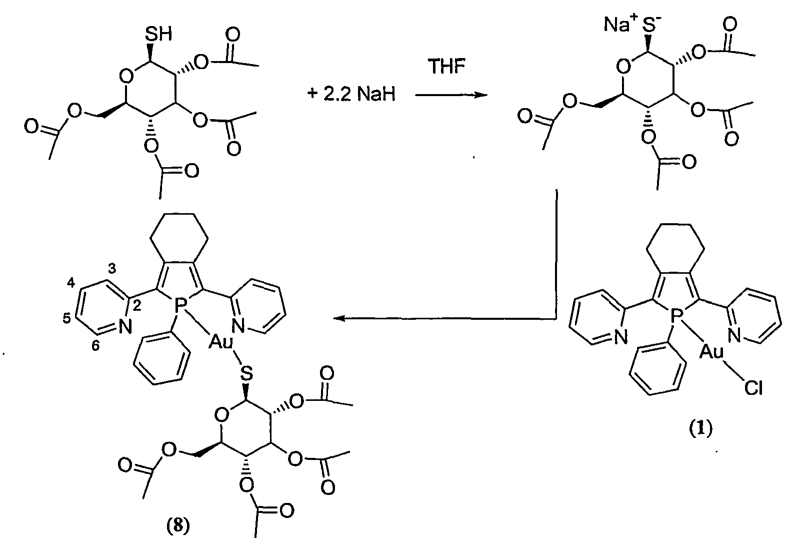

A une solution de 1-thio-β-D-glucose-tetraacetate (0,100 g ; 0,28 mmol) dans THF (10 mL) est ajouté 2,2 équivalents de NaH (0,014 g ; 0,61 mmol). La solution est laissée sous agitation pendant 90 minutes, puis un équivalent du complexe (1) (0,165 g ; 0,028 mmol) est additionné. Le mélange résultant est laissé sous agitation pendant 90 minuties. La solution est concentrée au quart sous vide, puis de l'éther (20 mL) est ajouté. L'agitation est poursuivie pendant 10 minutes. Le surnageant est recueilli et le résidu solide est extrait une nouvelle fois avec de l'éther (20 mL). Les phases liquides sont réunies et les solvants sont éliminés sous vide. Le complexe (8) est alors purifié par chromatographie sur colonne de gel de silice avec le mélange éther/acétate d'éthyle (65/35) comme éluant. Le complexe (8) est obtenu sous la forme d'une poudre jaune avec un rendement de 62%.
¹H NMR (CDCl₃, 200 MHz): δ = 1.77 (s, 3H, C*H*₃ sucre), 1.73-1.88 (m, 4H, =CCH₂C*H*₂), 1.99 (s, 3H, C*H*₃ sucre), 2.00 (s, 3H, C*H*₃ sucre), 2.02 (s, 2H, C*H*₂ sucre), 2.07 (s, 3H, C*H*₃ sucre), 2.91-3.10 (m, 2H, =CCH₂), 3.22-3.42 (m, 2H, =CCH₂), 3.64-3.74 (m, 1H, C*H* sucre), 3.95-4.16 (m, 2H, C*H* sucre), 4.88-5.2 (m, 2H, C*H* sucre), 7.03-7.15 (m, 2H, H⁵ Py), 7.23-7.31 (m, 3H, H_{méta} Ph, Hₚₐᵣₐ Ph), 7.58-7.81 (m, 6H, H⁴ Py ,H³ Py, Hₒᵣₜₕₒ Ph), 8.53 (d large, *J*_{HH} = 4.7 Hz,1H, H⁶ Py), 8.60 (dl, *J*_{HH} = 4.7 Hz,1H, H⁶ Py).
¹³C{¹H}NMR (CD₂Cl₂, 75.469 MHz): δ = 20.4 (s, *C*H₃ sucre), 22.3 (s, =CCH₂*C*H₂), 29.7 (s, =C*C*H₂), 29.8 (s, =C*C*H₂), 29.9 (s1, =C*C*H₂), 62.7 (s, C sucre), 68.8 (s, C sucre), 74.1 (s, C sucre), 75.7 (s, C sucre), 77.6 (s, C sucre), 83.0 (s, C sucre), 122.2 (s, C⁵ Py), 122.3 (s, C⁵ Py), 123.7 (d, *J*_{PC} = 6.4 Hz, C³ Py), 123.9 (d, *J*_{PC} = 6.3 Hz, C³ Py), 128.7 (s, *m*-Ph), 128.9 (s, *m*-Ph), 131.3 (s, *p*-Ph), 131.4 (s, *p*-Ph), 133.9 (s, *o-*Ph), 134.1 (s, *o*-Ph), 136.5 (s1, C⁴ Py), 149.2 (s, C⁶ Py), 149.3 (s, C⁶ Py), 152.1 (s, C² Py), 152.3 (s, C² Py), 153.01 (d, *J*_{PC} = 6.3 Hz, P-C=*C*), 153.46 (d, *J*_{PC} = 6.1 Hz, P-C=*C*), 169.3 (s, C=O sucre), 169.4 (s, C=O sucre), 169.9 (s, C=O sucre), 170.4 (s, C=O sucre).
³¹P{¹H} NMR (CDCl₃, 81.0 MHz) δ= +47.3 ppm (s large);
HR-MS (ESI, CH₂Cl₂): *m*/*z* 951.1768 (M⁺+Na⁺: calculé 951.1755)

### Exemple 6

### Etude des propriétés d'inhibition de la GR et de la TrxR humaines par les composés (1) à (4)

Les dérivés phqspholes contenant de l'or et du platine ont identifiés comme des inhibiteurs puissants de la GR et de la TrxR humaines (voir **Tableau 1**). Avec soit le substrat TrxC72S, ou le DTNB, de très basses valeurs d'IC50 dans le bas nanomolaire - réaction quasi- stoechiométrique - ont été obtenues avec la TrxR humaine type sauvage. Par contre, les valeurs d'IC50 avec le mutant Sec → Cys étaient au moins 1000 fois plus hautes prouvant que le résidu sélénocystéine est la cible d'inhibition de la TrxR humaine. La GR humaine était très efficacement inhibitée par les complexes de l'or et moins efficacement par ceux de platine (voir **Tableau 1**). Une réduction de ces enzymes est nécessaire pour compléter l'inhibition, ce qui prouve que le site actif composé des résidus Cys/Cys dans la GR ou Cys/Sec dans la TrxR est le site de l'inactivation. La pré-réduction de ces enzymes accélère l'inhibition. La réaction entre l'inhibiteur et ces enzymes est très rapide et pas exactement mesurable avec les techniques d'enzymologie standard. Les inhibiteurs montrent une très faible compétition avec le GSSG et la Trx avec des valeurs de Ki dans l'échelle du bas µM. Cette compétition ne contribuerait vraisemblablement pas avec l'inactivation irréversible enzymatique.

La stoechiométrie de liaison de (1) à la GR humaine a été déterminée par titration. Basé sur ces expériences, (1) se lie très étroitement au site de liaison du GSSG de chaque sous-unité de GR humaine. La valeur de la constante de dissociation Kd = Ki = [GR humaine][(1)]/[GR humaine*(1)], déterminée à partir des cinétiques mesurées en vitesse initiale avec 0.5-2.0 µM GSSG, était de 0.46 µM. Les différences du coefficient d'extinction à des longueurs d'onde sélectionnées ont révélé une stoechiométrie de 1.25 équivalents de (1) par FAD ou par sous-unité de GR humaine. Bien que de nombreux inhibiteurs hydrophobes de la GR humaine et de la GR de *Plasmodium falciparum* se lient dans la cavité entre les deux sous-unités du dimère avec une stoechiométrie de 0.5 par sous-unité, ce n'est pas le cas pour l'inhibiteur (1). La stoechiométrie de 1.25 équivalents de (1) par FAD ou par sous-unité de GR humaine peut s'expliquer par la présence d'une seconde molécule de (1) liée moins étroitement à une autre position dans la GR humaine, en confirmation avec la structure 3D du complexe GR-(1). La structure tridimensionnelle de la GR humaine alkylée montre en effet deux sites de liaison pour le métallo-phosphole (1) :
- l'atome d'or (Au) est covalemment lié entre la Cys58 et la Cys63 qui forment le centre rédox du site actif. Le mécanisme d'inactivation de la GR humaine implique donc une coordination S-Au(I)-S et la perte du ligand phosphole ;
- le second site pour le métallo-phosphole (1) est la Cys284 exposée au solvant, à 23 Å du site actif et localisé dans une cavité hydrophobe. Dans ce cas, l'atome de soufre de la Cys284 reste lié à l'entité Au-phosphole.

Basées sur des études de cinétique aussi bien que sur les données préliminaires de la radiocristallographie, (1) réagit avec la Cys58 et/ou la Cys63 de la GR humaine selon l'ordre séquentiel de l'ajout des réactants dans le milieu de pré-incubation, i.e. pré-réduction de l'enzyme par l'agent réducteur puis réaction avec l'inhibiteur, ou pré-réaction de l'enzyme avec l'inhibiteur puis réduction par l'agent réducteur. Quelque soit l'ordre séquentiel choisi, les molécules de GR humaine modifiées par (1) n'ont plus d'activité catalytique. En conclusion, (1) se lie étroitement au site de liaison du GSSG de chaque sous-unité de GR humaine (Eox) avec un Kd dans l'échelle submicromolaire. De plus, l'inhibiteur forme une liaison covalente avec la Cys58 et/ou la Cys63 de l'enzyme réduite, ce qui conduit à une inactivation complète de la GR humaine.

En se basant sur les fonctions essentielles de la TrxR et de la GR humaines dans l'équilibre redox cellulaire, la promotion de la croissance cellulaire, la synthèse de l'ADN, les processus de régulation redox-dépendants aussi bien que la résistance aux médicaments, ces protéines servent de cibles prometteuses pour le développement des agents chimiothérapeutiques contre les tumeurs, les parasites, les insectes, et autres maladies caractérisées par une inflammation et/ou une intense prolifération cellulaire (comme la polyarthrite rhumatoïde, le syndrome de Sjögren, le psoriasis). Les phospholes (1) à (4) aussi bien que leurs dérivés sont hautement efficaces comme inhibiteurs de TrxR et GR humaines et peuvent donc servir en tant que médicaments potentiels pour la chimiothérapie des maladies mentionnées ci-dessus aussi bien que pour étudier le métabolisme cellulaire redox.

Dans les expériences préliminaires, les inhibiteurs sont actifs contre les cellules humaines des glioblastomes dans l'échelle du bas micromolaire.

### Exemple 7

### Tests de prolifération in vitro du complexe (1) et analogues (2)-(6) et (8)

Le Kit III BrdU Marquage et Détection (Roche Diagnostics, Mannheim, Germany) a été utilisé pour déterminer la synthèse d'ADN de différentes lignées cellulaires de glioblastomes humains (NCH37, NCH82 et NCH 89) en présence des différents composés (1-8).

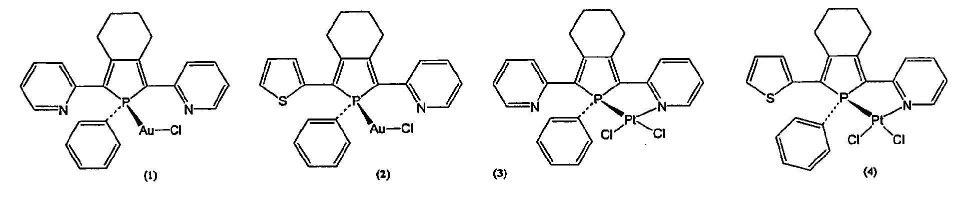

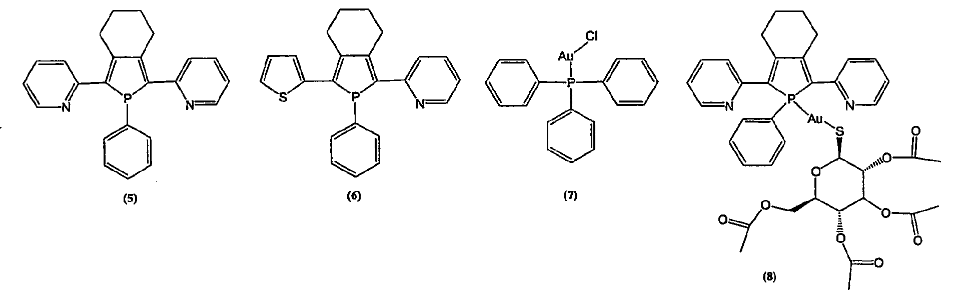

Brièvement, les cellules tumorales sont mises en culture et dupliquées en plaques 96-puits dans le milieu RPMI 1640 complété avec 10% de sérum de veau fétal et des antibiotiques (densité cellulaire: 7 x 10³ cellules). Après 24 h les composés (1)-(8) ont été ajoutés à différentes concentrations. Après 48 h, le réactif 5-bromo-2'-deoxyuridine (BrdU) a été ajouté à la concentration finale de 10 µM pour une période d'incubation de 19 h. La valeur moyenne de l'absorbance des échantillons de contrôles ne contenant pas de composés (1)-(8) est définie à 100% comme valeur maximale de prolifération. Le composé (7) (complexe chlorure Au-triphenylphosphine, (PPh₃)AuCl) et la nitrosourée 1,3-bis(2-chloroéthyl)-1-nitrosourea (BCNU, carmustine) sont des contrôles positifs déjà reportés dans la littérature. Les différentes lignées cellulaires de glioblastomes testées sont résistantes à la carmustine, qui est un médicament couramment utilisé en thérapie anticancéreuse, comme agent cytostatique contre les tumeurs de cerveau.

Sous ces conditions, les effets anti-prolifératifs les plus puissants parmi les dérivés (1)-(8) sont observés (**Tableau 2**), après traitement, pour le complexe (8), suivi du composé (1), du composé (2), et du composé (7) ((PPh₃)AuCl) avec des valeurs d'IC₅₀ dans l'échelle du bas micromolaire. Les composés (5) et (6) présentent également un effet anti-prolifératif supérieur à celui du composé (7) sur la lignée cellulaire NCH89. Par comparaison, la carmustine a montré 50% d'inhibition de la croissance des lignées cellulaires de glioblastomes NCH37, NCH82, et NCH89 à 300 ± 12, 385 ± 25, et 615 ± 38µM, respectivement.

### Exemple 8

### Activité antitumorale du complexe (1) in vivo chez le rat

L'activité antitumorale du complexe a été testée chez le rat. Le modèle d'inoculation orthopique de cellules du gliome de rat C6 est un modèle *in vivo* bien établi pour analyser et évaluer les effets thérapeutiques de molécules contre les gliomes malins.

Les cellules de gliome C6 de rat ont été implantées dans le cerveau de rats Wistar mâles âgés de 6 à 8 semaines. Les têtes de rats ont été fixées dans un cadre d'orientation stéreotactique, et 5 x 10⁵ cellules tumorales ont été inoculées (de façon latérale à 4 mm à partir du bregma et à une profondeur de 5-6 mm sous la dure-mère). Les tailles des tumeurs dans les cerveaux de rats ont été déterminées en imagerie par résonance magnétique (RMI) conventionnelle. Celle-ci a été effectuée aux jours 9 et 15 avec une unité Small-Bore RMI 2.35 Tesla. Avant imagerie, un agent paramagnétique de contraste a été injecté en route intraveineuse à la dose de 0.1 mL par 100 g de masse corporelle. Sur la base des images RMI conventionnelles, toutes les tumeurs ont pu être bien délimitées du tissu cérébral entourant la tumeur.

Après confirmation de la croissance tumorale au jour 9 par RMI, les rats porteurs de tumeurs ont été traités en injection intraveineuse avec le composé (1) à une concentration de 25 mg/kg (n = 4). La croissance tumorale a été comparée à celle des animaux contrôles non traités (n = 4). Le traitement a démarré au jour 9 et continué aux jours 11 et 13. Au jour 15 après l'implantation des cellules tumorales, tous les rats ont été sacrifiés par décapitation. Dans ces conditions, une réduction de la croissance tumorale de 44% a été observée entre les jours 9 et 15 à la dose du composé (1) de 25 mg /kg de masse corporelle comparé avec celle du groupe contrôle (**Figure 1****).**

La masse corporelle totale des rats C6 porteurs de gliomes n'a montré aucune différence significative entre les deux groupes d'animaux traités ou contrôles. De même, aucun animal n'est mort par traitement avec le composé (1) indiquant que la thérapie a été bien tolérée à 25 mg/kg.

### Exemple 9

### Liaison à l'ADN du complexe 1

En plus de son activité majeure sur les disulfure réductases humaines, la glutathion réductase et la thioredoxine réductase, la liaison du complexe (1) à l'ADN a été mesurée dans des tests de dénaturation thermique de l'ADN. Le complexe 1 s'est révélé être actif dès 1 µM dans les essais réalisés à une concentration comprise entre 0 et 20 µM de complexe (1).

**Tableau 1. Inhibition des TrxR et GR humaines par les phospholes P9-P12. Conditions : 10 min de pre-incubation avec 200 µM NADPH (dans l'essai de réduction du DTNB par la TrxR) ou avec 100 µM NADPH (dans l'essai de réduction de la Trx par la TrxR, et l'essai de réduction du GSSG par la GR). Concentrations enzymatiques: avec le mutant hTrxR-Mut, essai DTNB: 90.6 nM; essai Trx: 1.8 µM; avec le type sauvage hTrxR-Wt, essai DTNB: 4.8 nM, essai Trx: 24 nM. Les essais TrxR ont été réalisés soit en présence de 3 mM DTNB ou de 20 µM hTrxC72S; les essais GR ont été réalisés en présence de 100 µM GSSG.**

| **Inhibiteur** | **IC₅₀ WT hTrxR 3 mM DTNB/ 20 µM hTrxC72S** | **IC₅₀ hTrxRSec→Cys 3 mM DTNB/ 20 µM hTrxC72S** | **IC₅₀ GR humaine** |
|---|---|---|---|
| (1) | 0.8 nM/7 nM | 2500 nM/ 800 nM | 1 nM |
| (2) | 1 nM/7 nM | 2500 nM 1400 nM | 2 nM |
| (3) | 1 nM/7nM | 30000 nM/ 1400 nM | 1000 nM |
| (4) | 1 nM/8 nM | 24000 nM/ 2300 nM | 400 nM |

**Tableau 2. Effets cytotoxiques des composés 1- 8 sur la prolifération des lignées cellulaires de glioblastomes in vitro.**

| **Lignées** | **IC₅₀ (µM) des composés** | | | | | | | |
|---|---|---|---|---|---|---|---|---|
| | **1** | **2** | **3** | **4** | **5** | **6** | **7** | **8** |
| **NCH37** | 5,4±0,7 | 5,7±0,2 | 33±3,2 | 28,1±0,8 | nd | nd | 6,1±0,2 | nd |
| **NCH82** | 12,5±0,8 | 7,5±0,04 | 65±6,3 | 81,8±4,1 | nd | nd | 7,2±0,2 | 0,93±0,06 |
| **NCH89** | 10,8±0,8 | 15,2±0,4 | 43±3,7 | 78,4±3,1 | 6,7±0,3 | 8,5±0,5 | 20,3±0,7 | 2,54±0,45 |

| | | | | | | | | |
|---|---|---|---|---|---|---|---|---|
| nd, non déterminé | | | | | | | | |

### Références

Arnér ES, Holmgren A. Physiological functions of thioredoxin and thioredoxin reductase. Eur J Biochem 2000; 267:6102-09.
Arnér ES, Nakamura H, Sasada T, Yodoi J, Holmgren A, Spyrou G. Analysis of the inhibition of mammalian thioredoxin, thioredoxin reductase. and glutaredoxin by cis-diamminedichlorplatinum(II) and its major metabolite, glutathione-platinum complex. Free Rad Biol Med 2001; 31:1170-78.
Arscott LD, Gromer S, Schirmer RH, Becker K, Williams CH, Jr. The mechanism of thioredoxin reductase from human placenta is similar to the mechanisms of lipoamide dehydrogenase and glutathione reductase and is distinct from the mechanism of thioredoxin reductase from Escherichia coli. Proc Natl Acad Sci U S A 1997; 94:3621-26.
Becker K, Gromer S, Schirmer RH, Muller S. Thioredoxin reductase as a pathophysiological factor and drug target. Eur J Biochem 2000; 267:6118-25.
Becker K, Herold-Mende C, Park JJ, Lowe G, Schirmer RH. Human thioredoxin reductase is efficiently inhibited by (2,2':6',2"-terpyridine)platinum(II) complexes. Possible implications for a novel antitumor strategy. J Med Chem 2001; 44: 2784-92.
Becker K, Savvides S, Keese M, Schirmer RH & Karplus PA. Enzyme inactivation through sulfhydryl oxidation by physiologic NO-carriers. Nature Struct Biol 1998; 5: 267-271.
Davioud-Charvet E, Delarue S, Biot Ch, Schwöbel B, Boehme CC, Müssigbrodt A, Maes L, Sergheraert Ch, Grellier P, Schirmer RH, Becker K. A prodrug form of a Plasmodium falciparum glutathione reductase inhibitor conjugated with a 4-anilinoquinoline. J Med Chem 2001; 44: 4268-4276.
Davioud-Charvet E, McLeish MJ, Veine DM, Giegel D, Arscott LD, Andricopulo AD, Becker K, Müller S, Schirmer RH, Williams Jr CH, Kenyon GL. Mechanism-based inactivation of thioredoxin reductase by Mannich bases. Implication for cytotoxicity. Biochemistry 2003; 42, 13319-13330.
Dimmock JR, Vashishtha SC, Quail JW, Pugazhenthi U, Zimpel Z, Sudom AM, Allen TM, Kao GY, Balzarini J, De Clercq E. 4-(beta-Arylvinyl)-3-(beta-arylvinylketo)-1-ethyl-4-piperidinols and related compounds: a novel class of cytotoxic and anticancer agents. J. Med. Chem. 1998, 41, 4012-4020.
Engman L, Al-Maharik N, McNaughton M, Birmingham A, Powis G. Thioredoxin reductase and cancer cell growth inhibition by organotellurium antioxidants. Anticancer Drugs 2003; 14:153-61.
Fave, C. Thèse de Doctorat de l'Université de Rennes 1, n° 2900, octobre 2003.
Gladyshev VN, Jeang KT, Stadtman TC. Selenocysteine, identified as the penultimate C-terminal residue in human T-cell thioredoxin reductase, corresponds to TGA in the human placental gene. Proc Natl Acad Sci USA 1996; 93:6146-51.
Gromer S, Arscott LD, Williams CH, Jr., Schirmer RH, Becker K. Human placenta thioredoxin reductase. Isolation of the selenoenzyme, steady state kinetics, and inhibition by therapeutic gold compounds. J Biol Chem 1998; 273:20096-101.
Gromer S, Gross JH. Methylseleninate is a substrate rather than an inhibitor of mammalian thioredoxin reductase. Implications of the antitumor effects of selenium. J Biol Chem 2002; 277:9701-06.
Gromer S, Schirmer RH, Becker K. The 58 kDa mouse selenoprotein is a BCNU-sensitive thioredoxin reductase. FEBS Lett 1997; 412:318-20.
Gromer S, Wissing J, Behne D, et al. A hypothesis on the catalytic mechanism of the selenoenzyme thioredoxin reductase. Biochem J 1998; 332:591-92.
Hay, C., Hissler, M., Fischmeister, C., Rault-Berthelot, J., Toupet, L., Nyulaszi, L., Réau, R. Phosphole-containing p-conjugated systems: from model molecules to polymer films on electrodes. Chem. Eur. J., 2001, 7, 4222-4236.
Holmgren A. Antioxidant function of thioredoxin and glutaredoxin systems. Antioxid Redox Signal 2000; 2:811-20. Husbeck B, Powis G. The redox protein thioredoxin-1 regulates the constitutive and inducible expression of the estrogen metabolizing cytochromes P450 1B1 and 1A1 in MCF-7 human breast cancer cells. Carcinogenesis 2002; 23:1625-30.
Irmler, A., Bechthold, A., Davioud-Charvet, E., Hofmann, V., Réau, R., Gromer, S., Schirmer, R. H., and Becker, K. (2002) Disulfide reductases - Current developments, pp 803-815. In Flavins and Flavoproteins 2002. Chapman, S.K., Perham, R.N., Scrutton N.S., Eds. Agency for Scientific Publications, Berlin, 2002.
Kahlos K, Soini Y, Saily M, Koistinen P, Kakko S, Paakko, P, Holmgren A, Kinnula VL. Up-regulation of thioredoxin and thioredoxin reductase in human malignant pleural mesothelioma. Int J Cancer 2001; 95:198-204.
Kim MR, Chang HS, Kim BH, Bask SH, Lee SR, Kim JR. Involvements of mitochondrial thioredoxin reductase (TrxR2) in cell proliferation. Biochem Biophys Res Comm 2003; 304:119-24.
Lin, S, Del Razo LM, Styblo M, Wang C, Cullen WR, Thomas DJ. Arsenicals inhibit thioredoxin reductase in cultured rat hepatocytes. Chem Res Toxicol 2001; 14:305-11.
Lincoln DT, Ali Emadi EM, Tonissen KF, Clarke FM. The thioredoxin-thioredoxin reductase system: over-expression in human cancer. Anticancer Res 2003; 23:2425-33.
May JM, Morrow JD, Burk RF. Thioredoxin reductase reduces lipid hydroperoxides and spares alpha-tocopherol. Biochem Biophys Res Commun 2002; 292:45-49.
Nelsen, L. Rapport de DEA Chimie moléculaire, Université de Rennes 1, juin 2002.
Powis G, Kirkpatrick DL, Angulo M, Baker A. Thioredoxin redox control of cell growth and death and the effects of inhibitors. Chem Biol Interact 1998; 111-112:23-34.
Ross SA, Carr CA, Briet JW, Lowe G. Transfer of 4'-chloro-2,2':6',2"-terpyridine platinum(II) between human serum albumin, glutathione and other thiolate ligands. A possible selective natural transport mechanism for the delivery of platinum(II) drugs to tumour cells. Anticancer Drug Des 2000; 15:431-39.
Sandalova T, Zhong L, Lindqvist Y, Holmgren A, Schneider G. Three-dimensional structure of a mammalian thioredoxin reductase: Implications for mechanism and evolution of a selenocysteine-dependent enzyme. Proc Natl Acad Sci U S A 2001; 98:9533-38.
Sarma GN, Savvides SN, Becker K, Schirmer M, Schirmer RH & Karplus PA. Glutathione reductase of the malarial parasite Plasmodium falciparum: Crystal structure and inhibitor development. J Mol Biol 2003; 308: 893-907.
Sauthier, M., Leca, F., Toupet, L., Réau, R. Palladium Complexes of a novel family of P,N-chelates, the 2-(2-pyridyl)phospholes: synthesis, structural characterization, and catalytic activity for olefin/CO copolymerization. Organometallics, 2002, 21, 1591-1602.
Savvides SN, Scheiwein M, Boehme CC, Arteel GE, Karplus PA, Becker K & Schirmer RH. Crystal structure of the antioxidant enzyme glutathione reductase inactivated by peroxynitrite. J Biol Chem 2002; 277: 2779-2784.
Smith AD, Guidry CA, Morris VC, Levander OA. Aurothioglucose inhibits murine thioredoxin reductase activity in vivo. J Nutr 1999; 129:194-98.
Soini Y, Kahlos K, Napankangas U, Kaarteenaho-Wiik R, Saily M, Koistinen P, Paaakko P, Holmgren A, Kinnula VL. Widespread expression of thioredoxin and thioredoxin reductase in non-small cell lung carcinoma. Clin Cancer Res 2001; 7:1750-57.
Tamura T, Stadtman TC. A new selenoprotein from human lung adenocarcinoma cells: purification, properties, and thioredoxin reductase activity. Proc Natl Acad Sci U S A 1996; 93:1006-11.
Welsh SJ, Bellamy WT, Briehl MM, Powis G. The redox protein thioredoxin-1 (Trx-1) increases hypoxia-inducible factor lalpha protein expression: Trx-1 overexpression results in increased vascular endothelial growth factor production and enhanced tumor angiogenesis. Cancer Res 2002; 62:5089-95.
Williams CHJ. Lipoamide dehydrogenase, glutathione reductase, thioredoxin reductase, and mercuric ion reductase - a family of flavoenzyme transhydrogenases. In: Müller F, ed. Chemistry and biochemistry of flavoenzymes. Vol. 3. Boca Raton: CRC Press, 1992:121-211.
Wipf P, Hopkins TD, Jung JK, Rodriguez S, Birmingham A, Southwick EC, Lazo JS, Powis G. New inhibitors of the thioredoxin-thioredoxin reductase system based on a naphtoquinone spiroketal natural product lead. Bioorg Med Chem Lett 2001; 11:2637-41.
Xia L, NordmanT, Olsson JM, Damdimopoulos A, Bjorkhem-Bergman L, Nalvarte I, Eriksson LC, Amer ES, Spyrou G, Bjornstedt M. The mammalian cytosolic selenoenzyme thioredoxin reductase reduces ubiquinone. A novel mechanism for defense against oxidative stress. J Biol Chem 2003; 278:2141-46.
Zhao R, Holmgren A. A novel antioxidant mechanism of ebselen involving ebselen diselenide, a substrate of mammalian thioredoxin and thioredoxin reductase. J Biol Chem 2002; 277:39456-62.
Zhao R, Masayasu H, Holmgren A. Ebselen: a substrate for human thioredoxin reductase stongly stimulating its hydroperoxide reductase activity and a superfast thioredoxin oxidant. Proc Natl Acad Sci USA 2002; 99:8579-84.
Zhong L, Arnér ES, Holmgren A. Structure and mechanism of mammalian thioredoxin reductase: the active site is a redox-active selenolthiol/selenenylsulfide formed from the conserved cysteine-selenocysteine sequence. Proc Natl Acad Sci U S A 2000; 97:5854-59.
Zhong L, Arnér ES, Ljung J, Aslund F, Holmgren A. Rat and calf thioredoxin reductase are homologous to glutathione reductase with a carboxyl-terminal elongation containing a conserved catalytically active penultimate selenocysteine residue. J Biol Chem 1998; 273:8581-91.
Zhong L, Holmgren A. Mammalian thioredoxin reductases as hydroperoxide reductases. Methods Enzymol 2002; 347:236-43.

## Revendications

1. Médicament, **caractérisée en ce qu'**il comprend à titre de composé actif au moins un composé de formule générale (A) suivante : 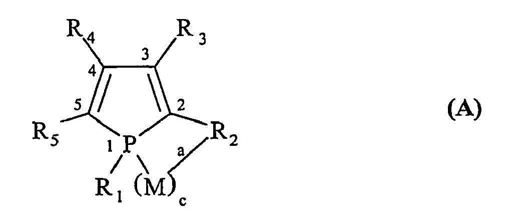 dans laquelle :
- a représente une liaison simple ou aucune liaison,
- c représente 0 lorsque M est absent ou 1 lorsque M est présent,
- R₁ représente un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- le cas échéant (b = 1) M représente un atome métallique, portant éventuellement de 1 à 2 substituants, R₆ et R₇, identiques ou différents, lesquels substituants sont choisis parmi
• un atome d'halogène,
• un groupement thio-osidique de 3 à 60 atomes de carbone, éventuellement substitué par un ou plusieurs groupements, identiques ou différents, choisis parmi les groupements protecteurs de groupements hydroxyles ou amines, tel qu'un groupement alcanoyle de 2 à 6 atomes de carbone, ou un groupement arylcarbonyle de 6 à 14 atomes de carbone,
• un groupement peptidique comprenant de 1 à 5 acides aminés, l'un au moins des acides aminés comprenant un groupement thio, tel que la S-cystéine ou le S-glutathion, ou
• un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, éventuellement substitués par un ou plusieurs groupements, identiques ou différents, choisis parmi un atome d'halogène, un groupement alkyle de 1 à 6 atomes de carbone, éventuellement substitué par un ou plusieurs atomes d'halogène, ou un groupement aryle de 6 à 14 atomes de carbone ;
en association avec un véhicule pharmaceutiquement acceptable.

2. Médicament selon la revendication 1, **caractérisée en ce qu'**il comprend à titre de composé actif au moins un composé de formule générale (I) suivante : 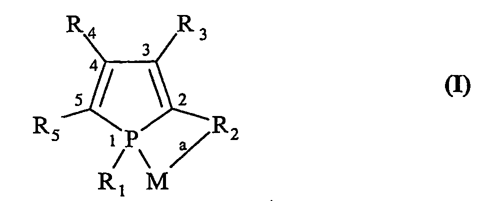 dans laquelle :
- a représente une liaison simple ou aucune liaison,
- R₁ représente un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- M représente un atome métallique, portant éventuellement de 1 à 2 substituants, R₆ et R₇, identiques ou différents, lesquels substituants sont choisis parmi
• un atome d'halogène,
• un groupement thio-osidique de 3 à 60 atomes de carbone, éventuellement substitué par un ou plusieurs groupements, identiques ou différents, choisis parmi les groupements protecteurs de groupements hydroxyles ou amines, tel qu'un groupement alcanoyle de 2 à 6 atomes de carbone, ou un groupement arylcarbonyle de 6 à 14 atomes de carbone, ou
• un groupement peptidique comprenant de 1 à 5 acides aminés, l'un au moins des acides aminés comprenant un groupement thio, tel que la S-cystéine ou le S-glutathion, ou
• un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, éventuellement substitués par un ou plusieurs groupements, identiques ou différents, choisis parmi un atome d'halogène, un groupement alkyle de 1 à 6 atomes de carbone, éventuellement substitué par un ou plusieurs atomes d'halogène, ou un groupement aryle de 6 à 14 atomes de carbone ;
en association avec un véhicule pharmaceutiquement acceptable.

3. Médicament selon la revendication 1 ou 2, **caractérisée en ce qu'**il comprend à titre de composé actif au moins un composé de formule générale (II) suivante : 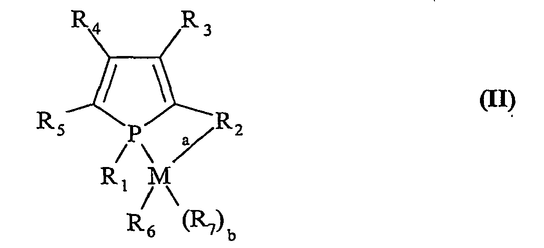 dans laquelle :
- a représente une liaison simple ou aucune liaison,
- b représente 0 ou 1,
- R₁, R₂, R₃, R₄ et R₅ sont tels que définis dans la revendication 1 ou 2,
- R₆ et R₇ représentent, indépendamment l'un de l'autre,
• un atome d'halogène,
• un groupement thio-osidique de 3 à 60 atomes de carbone, éventuellement substitué par un ou plusieurs groupements, identiques ou différents, choisis parmi les groupements protecteurs de groupements hydroxyles ou amines, tel qu'un groupement alcanoyle de 2 à 6 atomes de carbone, ou un groupement arylcarbonyle de 6 à 14 atomes de carbone, ou
• un groupement peptidique comprenant de 1 à 5 acides aminés, l'un au moins des acides aminés comprenant un groupement thio, tel que la S-cystéine ou le S-glutathion, ou
• un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, éventuellement substitués par un ou plusieurs groupements, identiques ou différents, choisis parmi un atome d'halogène, un groupement alkyle de 1 à 6 atomes de carbone, éventuellement substitué par un ou plusieurs atomes d'halogène, ou un groupement aryle de 6 à 14 atomes de carbone ;
- M représente un atome métallique divalent, trivalent ou tétravalent, sous réserve que lorsque M représente un atome métallique divalent alors a ne représente aucune liaison et b vaut 0, lorsque M représente un atome métallique trivalent alors a ne représente aucune liaison et b vaut 1 et que lorsque M représente un atome métallique tétravalent alors a représente une liaison simple et b vaut 1.

4. Médicament selon l'une des revendications 1 à 3, **caractérisée en ce qu'**il comprend à titre de composé actif au moins un composé de formule générale (III) : 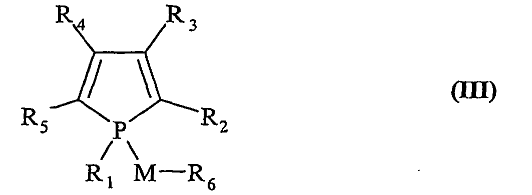 dans laquelle R₁, R₂, R₃, R₄, R₅ et R₆ sont tels que définis dans la revendication 1, 2 ou 3, et M représente un atome métallique divalent, tel que Au.

5. Médicament selon l'une des revendications 1 à 4, **caractérisée en ce qu'**il comprend à titre de composé actif au moins un composé de formule générale (IV) suivante : 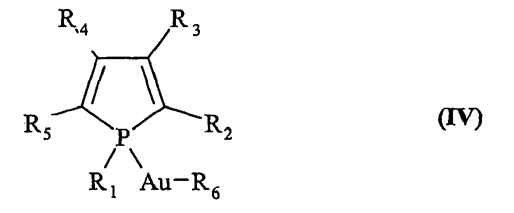 dans laquelle R₂, R₃, R₄, R₅ et R₆ sont tels que définis dans la revendication 1, 2 ou 3.

6. Médicament selon l'une des revendications 1 à 5, **caractérisée en ce qu'**il comprend à titre de composé actif au moins un composé de formule générale (IV') suivante : 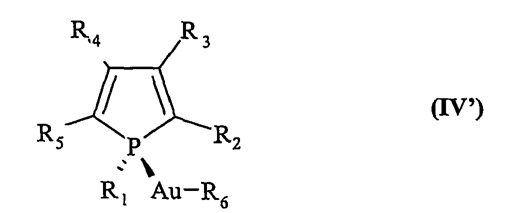 dans laquelle R₂, R₃, R₄, R₅ et R₆ sont tels que définis dans la revendication 1, 2 ou 3.

7. Médicament selon l'une des revendications 1 à 3, **caractérisée en ce qu'**il comprend à titre de composé actif au moins un composé de formule générale (V) : 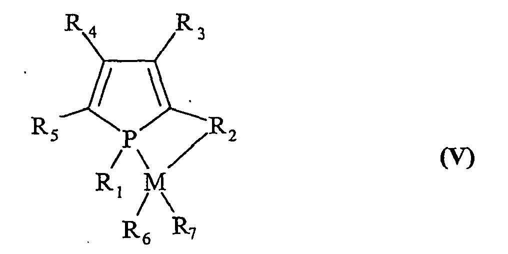 dans laquelle R₁, R₂, R₃, R₄, R₅, R₆ et R₇ sont tels que définis dans la revendication 1, 2 ou 3, et M représente un atome métallique tétravalent, tel que Pt.

8. Médicament selon l'une des revendications 1 à 3 et 7, **caractérisée en ce qu'**il comprend à titre de composé actif au moins un composé de formule générale (VI) suivante : 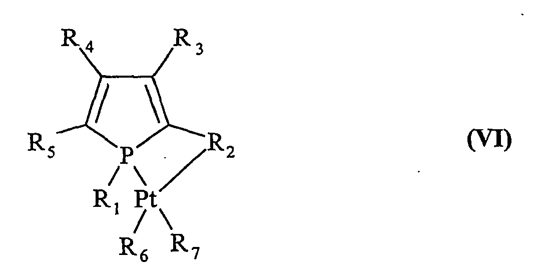 dans laquelle R₂, R₃, R₄, R₅ et R₆ sont tels que définis dans la revendication 1, 2 ou 3.

9. Médicament selon l'une des revendications 1 à 3, 7 et 8, **caractérisée en ce qu'**il comprend à titre de composé actif au moins un composé de formule générale (VI') suivante : 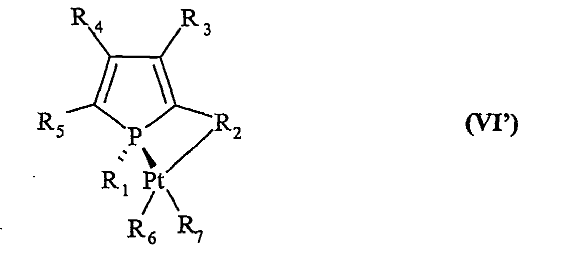 dans laquelle R₂, R₃, R₄, R₅ et R₆ sont tels que définis dans la revendication 1, 2 ou 3.

10. Médicament selon l'une des revendications 1 à 3 et 7 à 9, **caractérisée en ce qu'**il comprend à titre de composé actif au moins un composé de formule générale (VII) suivante : 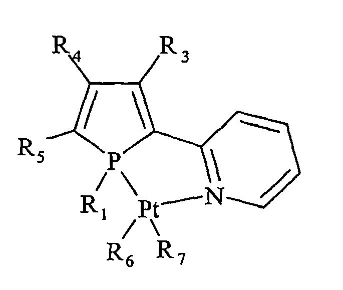 dans laquelle R₁, R₃, R₄, R₅, R₆ et R₇ sont tels que définis dans la revendication 1, 2 ou 3.

11. Médicament selon l'une des revendications 1 à 3 et 7 à 10, **caractérisée en ce qu'**il comprend à titre de composé actif au moins un composé de formule générale (VII') suivante : 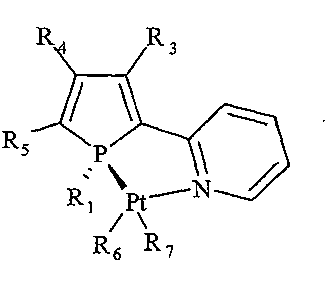 dans laquelle R₁, R₃, R₄, R₅, R₆ et R₇ sont tels que définis dans la revendication 1, 2 ou 3.

12. Médicament selon l'une des revendications 1 à 11, **caractérisée en ce que :**
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle , ou pyridyle , substitué ou non, et plus particulièrement un groupement phényle, 2-thiényle ou 2-pyridyle ;
- R₆ et le cas échéant R₇ représentent indépendamment l'un de l'autre un halogène, et plus particulièrement un atome de chlore, un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiopactose, le thiolactose hepta-acétate.

13. Médicament selon l'une des revendications 1 à 12, **caractérisée en ce qu'**il comprend à titre de composé actif au moins un composé de formule suivante : 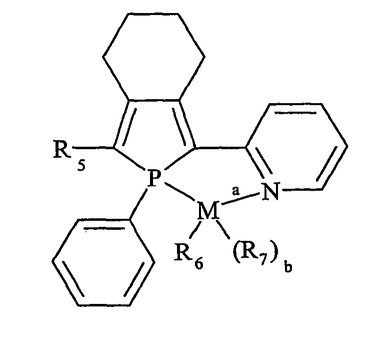 dans laquelle R₅ représente un groupe 2-thiényle ou 2-pyridyle, M représente Au ou Pt, a représente une simple liaison lorsque M représente Pt ou aucune liaison lorsque M représente Au, b représente 1 lorsque M représente Pt et 0 lorsque M représente Au, et R₆ et R₇ sont tels que définis dans la revendication 12.

14. Médicament selon la revendication 13, **caractérisée en ce qu'**il comprend à titre de composé actif au moins un composé de formule suivante : 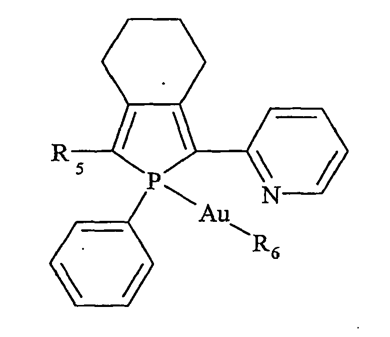 dans laquelle R₅ représente un groupe 2-thiényle ou 2-pyridyle et R₆ est tel que défini dans la revendication 12.

15. Médicament selon l'une des revendications 1 à 14, **caractérisée en ce qu'**il comprend à titre de composé actif au moins un composé de formule suivante :

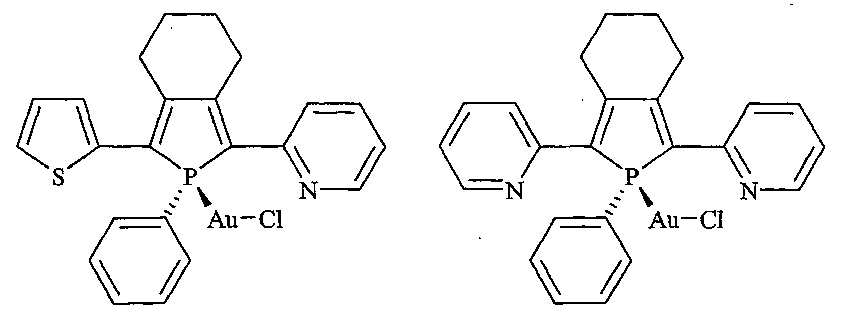

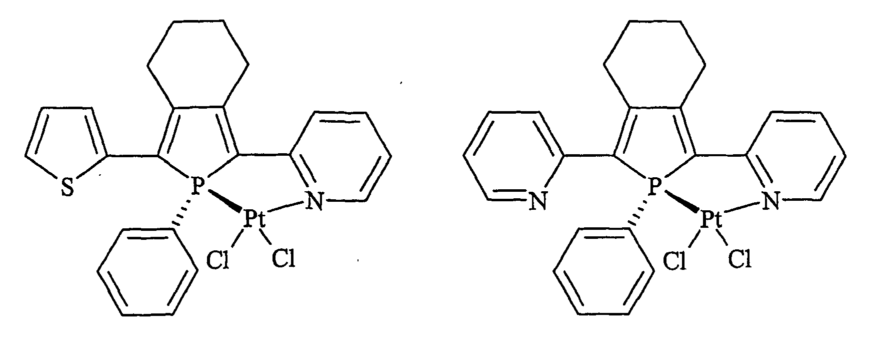

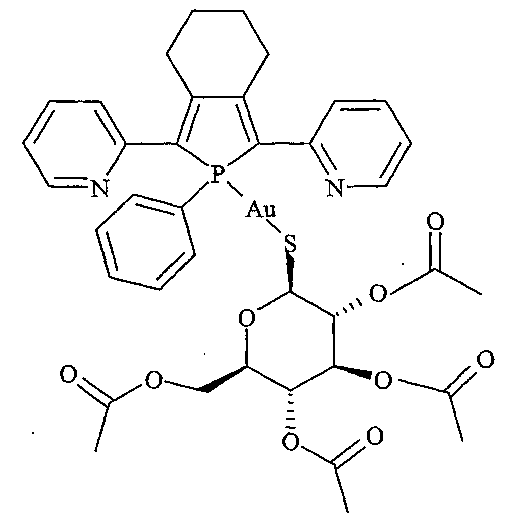

16. Médicament selon la revendication 1, **caractérisée en ce qu'**il comprend à titre de composé actif au moins un composé de formule générale (VIII) suivante : 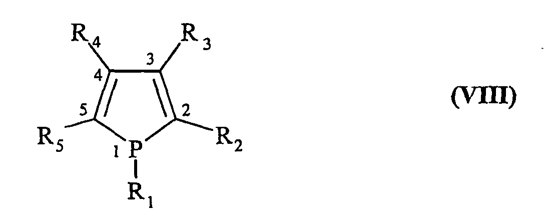 dans laquelle :
- R₁ représente un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, ou un groupement aryle de 6 à 14 atomes dé carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, ou un groupement aryle de 6 à 14 atomes de carbone ;
en association avec un véhicule pharmaceutiquement acceptable.

17. Médicament selon la revendication 16, **caractérisée en ce que :**
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle ou pyridyle substitué ou non, et plus particulièrement un groupement phényle, 2-thiényle ou 2-pyridyle.

18. Médicament selon la revendication 16 ou 17, **caractérisée en ce qu'**il comprend à titre de composé actif au moins un composé de formule suivante : 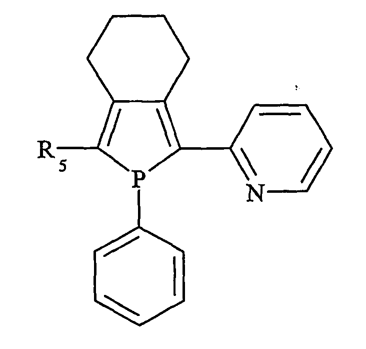 dans laquelle R₅ est tel que défini dans la revendication 17.

19. Médicament selon l'une des revendications 16 à 18, **caractérisée en ce qu'**il comprend à titre de composé actif au moins un composé de formule suivante :

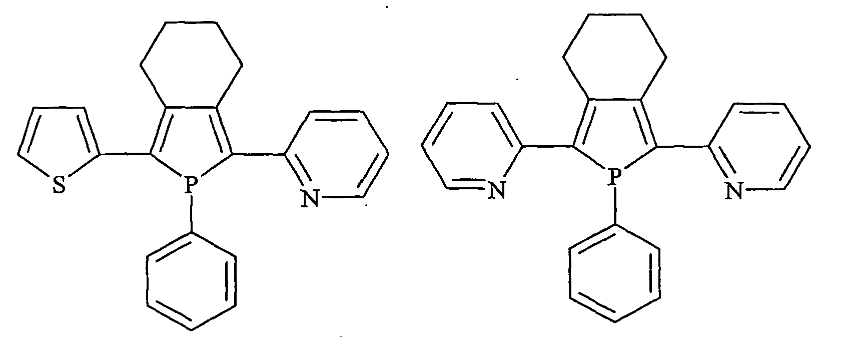

20. Médicament selon l'une des revendications 1 à 19, **caractérisée en ce qu'**il comprend à titre de composé actif au moins un composé de formule générale (A), et plus particulièrement de formule (I) à (VIII), en association avec au moins un composé anticancéreux tels que le cisplatin, la mitomycine C, la doxorubicine, l'étoposide, la carmustine.

21. Produits comprenant:
* au moins un composé de formule générale (A), et plus particulièrement de formule (I) à (VIII), tel que défini dans l'une des revendications 1 à 19,
* et au moins un composé anticancéreux, tel que défini dans la revendication 20,
comme produits de combinaison pour une utilisation simultanée, séparée ou étalée dans le temps, en thérapie cancéreuse.

22. Produit de combinaison selon la revendication 21, **caractérisé en ce qu'**il contient un produit de formule (A) et un agent anticancéreux dans un rapport d'environ 0,1/1 à environ 2,5/1 et, le cas échéant, un ou plusieurs véhicules pharmaceutiquement acceptables.

23. Médicament ou produits de combinaison, selon l'une des revendications 1 à 22, **caractérisés en ce qu'**ils se présentent sous une forme administrable par voie intraveineuse.

24. Médicament, ou produits de combinaison, selon l'une des revendications 1 à 23, **caractérisés en ce que** la posologie des composés de formule (A) contenus dans ces compositions pharmaceutiques ou produits de combinaison, est d'environ 5 à environ 200 mg /m²/j ou par cure.

25. Utilisation d'au moins un composé de formule générale (I) tel que défini dans l'une des revendications 2 à 15 pour la préparation d'un médicament destiné à la prévention ou au traitement de pathologies liées à une activité excessive de la glutathion réductase et/ou de la thiorédoxine réductase.

26. Utilisation selon la revendication 25, **caractérisée en ce que** les pathologies liées à une activité excessive de la glutathion réductase et/ou de la thiorédoxine réductase, sont :
- les cancers, à savoir toutes tumeurs liquides ou solides, et plus particulièrement les cancers associés à l'infection par le virus d'Epstein-Barr,
- la polyarthrite rhumatoïde,
- le psoriasis ou les rhumatismes psoriasiques,
- le syndrome de Sjögren,
- l'infection au virus HIV et maladies associées comme le carcinome de Kaposi.

27. Utilisation d'au moins un composé de formule (VIII) tel que défini dans l'une des revendications 16 à 19, pour la préparation d'un médicament destiné au traitement des cancers.

28. Utilisation d'au moins un composé de formule générale (A) tel que défini dans l'une des revendications 1 à 19, pour la préparation d'un médicament destiné à la prévention ou au traitement des phénomènes de résistance aux médicaments anticancéreux tels que le cisplatin, la mitomycine C, la doxorubicine, l'étoposide, la carmustine, et, le cas échéant, aux médicaments antinfectieux tels que le métronidazole, l'isoniazide.

29. Composé de formule générale (IX) suivante : 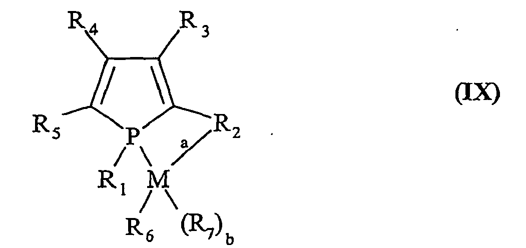 dans laquelle :
- a représente une liaison simple ou aucune liaison,
- b représente 0 ou 1,
- R₁ représente un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- R₂ représente un groupement hétéroaryle azoté ou un hétérocycle azoté à 5 ou 6 atomes possédant de 1 à 4 atomes d'azote ;
- R₃ et R₄ représentent, indépendamment l'un de l'autre, un atome d'hydrogène, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone, le cas échéant un groupement alkyle peut ponter les atomes de carbone en positions 3 et 4 du cycle phosphole portant, respectivement, les substituants R₃ et R₄, de manière à former un cycle de 3 à 8 atomes de carbone ;
- R₅ représente un atome d'hydrogène, un hétérocycle à cinq ou six chaînons possédant de 1 à 4 atomes d'azote ou de soufre, ou un groupement
• alkyle linéaire ou ramifié de 1 à 6 atomes de carbone,
• cycloalkyle saturé ou non saturé de 3 à 7 atomes de carbone,
• aryle de 6 à 14 atomes de carbone,
• alkoxy linéaire ou ramifié de 1 à 6 atomes de carbone,
• cycloalkoxy saturé ou non saturé de 3 à 7 atomes de carbone,
• aryloxy de 6 à 14 atomes de carbone,
• alkylthio linéaire ou ramifié de 1 à 6 atomes de carbone,
• cycloalkylthio saturé ou non saturé de 3 à 7 atomes de carbone, ou
• arylthio de 6 à 14 atomes de carbone,
éventuellement substitué par un ou plusieurs groupements, identiques ou différents, choisis parmi un atome d'halogène, un groupement alkyle linéaire ou ramifié de 1 à 6 atomes de carbone, éventuellement substitué par un ou plusieurs atomes d'halogène, ou un groupement aryle de 6 à 14 atomes de carbone ;
- M représente un atome métallique divalent, trivalent ou tétravalent, sous réserve que lorsque M représente un atome métallique divalent alors a ne représente aucune liaison et b vaut 0, lorsque M représente un atome métallique trivalent alors a ne représente aucune liaison et b vaut 1 et que lorsque M représente un atome métallique tétravalent alors a représente une liaison simple et b vaut 1,
- R₆ représente un groupement thio-osidique de 3 à 60 atomes de carbone, éventuellement substitué par un ou plusieurs groupements, identiques ou différents, choisis parmi les groupements protecteurs de groupements hydroxyles ou amines, tel qu'un groupement alcanoyle de 2 à 6 atomes de carbone, ou un groupement arylcarbonyle de 6 à 14 atomes de carbone,
- R₇ représente
• un atome d'halogène,
• un groupement thio-osidique de 3 à 60 atomes de carbone, éventuellement substitué par un ou plusieurs groupements, identiques ou différents, choisis parmi les groupements protecteurs de groupements hydroxyles ou amines, tel qu'un groupement alcanoyle de 2 à 6 atomes de carbone, ou un groupement arylcarbonyle de 6 à 14 atomes de carbone,
• un groupement peptidique comprenant de 1 à 5 acides aminés, l'un au moins des acides aminés comprenant un groupement thio, tel que la S-cystéine ou le S-glutathion, ou
• un groupement thioalkyle linéaire ou ramifié de 1 à 6 atomes de carbone, un groupement thiocycloalkyle saturé ou non saturé de 3 à 7 atomes de carbone ou un groupement thioaryle ou thiohétéroaryle de 6 à 14 atomes de carbone, éventuellement substitués par un ou plusieurs groupements, identiques ou différents, choisis parmi un atome d'halogène, un groupement alkyle de 1 à 6 atomes de carbone, éventuellement substitué par un ou plusieurs atomes d'halogène, ou un groupement aryle de 6 à 14 atomes de carbone.

30. Composé selon la revendication 29, de formule générale (X) : 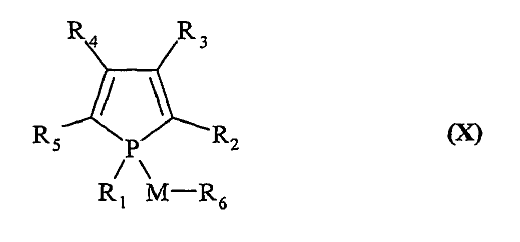 dans laquelle R₁, R₂, R₃, R₄, R₅ et R₆ sont tels que définis dans la revendication 29, et M représente un atome métallique divalent, tel que Au.

31. Composé selon la revendication 29 ou 30, de formule générale (XI): 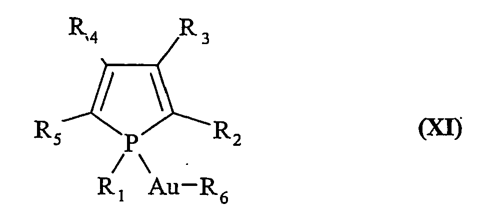 dans laquelle R₂, R₃, R₄, R₅ et R₆ sont tels que définis dans la revendication 26.

32. Composé selon l'une des revendications 29 à 31, dans lequel :
- R₁ représente un groupement cycloalkyle de 3 à 7 atomes de carbone ou aryle de 6 à 14 atomes de carbone, et plus particulièrement un groupement cyclohexyle ou phényle ;
- R₂ représente un groupement hétéroaryle azoté à six atomes possédant de 1 à 3 atomes d'azote, et plus particulièrement le groupement 2-pyridyle ;
- R₃ et R₄ représentent indépendamment l'un de l'autre un groupements alkyle de 1 à 6 atomes de carbone, et plus particulièrement un groupement alkyle à 3 ou 4 atomes de carbones reliant les atomes en positions 3 et 4 du cycle phosphole ;
- R₅ représente un groupement aryle de 6 à 14 atomes de carbone, thiényle , ou pyridyle , substitué ou non, et plus particulièrement un groupement phényle, 2-thiényle ou 2-pyridyle ;
- R₆ représente un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiolactose, le thiolactose hepta-acétate ;
- le cas échéant, R₇ représente un halogène, et plus particulièrement un atome de chlore, un thio-ose, notamment choisi parmi un thiopentose ou un thiohexose, ou un thiodioside, éventuellement substitué par un ou plusieurs groupements protecteurs de groupements hydroxyles ou amines, et plus particulièrement le thioglucose, le thioglucose tétraacétate, le thiomannose, le thiomannose tétraacétate, le thiogalactose, le thiogalactose tétraacétate, le thioribose, le thioribose triacétate, le thioxylose, le thioxylose triacétate, le thio-allose, le thio-allose tétraacétate, le thiotalose, le thiotalose tétraacétate, le thiofucose, le thiofucose tétraacétate, le thio-N-acétyl-glucosamine, thio-N-acétyl-glucosamine triacétate, le thio-N-acétyl-galactosamine, le thio-N-acétyl-galactosamine triacétate, le thio-N-acétyl mannosamine, le thio-N-acétyl mannosamine triacétate, le thiolactose, le thiolactose hepta-acétate.

33. Composé selon l'une des revendications 29 à 32, de formule suivante : 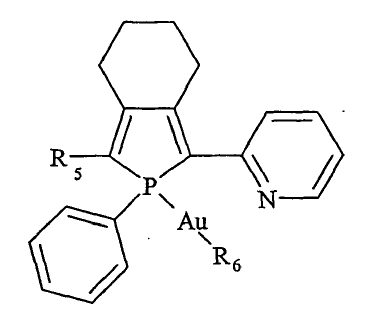 dans laquelle R₅ et R₆ sont tels que définis dans la revendication 32.

34. Composé selon l'une des revendications 29 à 33, de formule suivante :

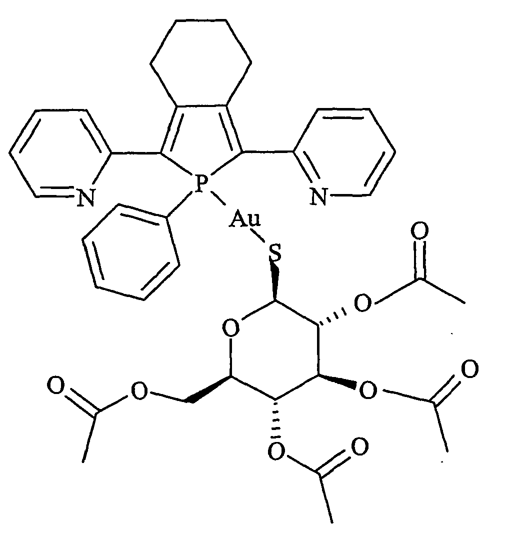

## Claims

1. A drug **characterized in that** it comprises as active ingredient at least one compound of general formula (A) below: 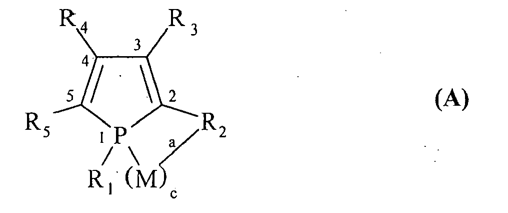 in which:
- a represents a single bond or no bond,
- c represents 0 when M is absent or 1 when M is present,
- R₁ represents a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- R₂ represents a nitrogen-containing heteroaryl group or a nitrogen-containing heterocycle with 5 or 6 atoms having from 1 to 4 nitrogen atoms;
- R₃ and R₄ represent, independently of one another, a hydrogen atom, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms, optionally an alkyl group can bridge the carbon atoms in positions 3 and 4 of the phosphole ring carrying, respectively, the substituents R₃ and R₄, so as to form a ring with 3 to 8 carbon atoms;
- R₅ represents a hydrogen atom, a heterocycle with five or six members having from 1 to 4 nitrogen or sulphur atoms, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- optionally (b = 1) M represents a metal atom, optionally carrying from 1 to 2 substituents, R₆ and R₇, identical or different, which substituents are chosen from
• a halogen atom,
• a thio-osidic group with 3 to 60 carbon atoms, optionally substituted by one or more groups, identical or different, chosen from the protective groups of the hydroxyl or amine groups, chosen from an alkanoyl group with 2 to 6 carbon atoms, or an arylcarbonyl group with 6 to 14 carbon atoms,
• a peptide group comprising from 1 to 5 amino acids, at least one of the amino acids comprising a thio group, chosen from S-cysteine or S-glutathione, or
• a linear or branched thioalkyl group with 1 to 6 carbon atoms, a saturated or unsaturated thiocycloalkyl group with 3 to 7 carbon atoms or a thioaryl or thioheteroaryl group with 6 to 14 carbon atoms, optionally substituted by one or more groups, identical or different, chosen from a halogen atom, an alkyl group with 1 to 6 carbon atoms, optionally substituted by one or more halogen atoms, or an aryl group with 6 to 14 carbon atoms;
in combination with a pharmaceutically acceptable vehicle.

2. The drug according to claim 1, **characterized in that** it comprises as active ingredient at least one compound of general formula (I) below: 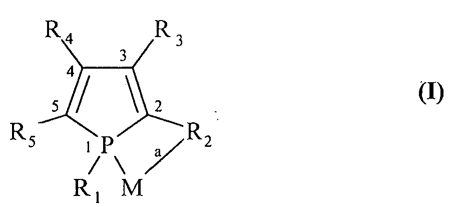 in which:
- a represents a single bond or no bond,
- R₁ represents a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- R₂ represents a nitrogen-containing heteroaryl group or a nitrogen-containing heterocycle with 5 or 6 atoms having from 1 to 4 nitrogen atoms;
- R₃ and R₄ represent, independently of one another, a hydrogen atom, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms, optionally an alkyl group can bridge the carbon atoms in positions 3 and 4 of the phosphole ring carrying, respectively, the substituents R₃ and R₄, so as to form a ring with 3 to 8 carbon atoms;
- R₅ represents a hydrogen atom, a heterocycle with five or six members having from 1 to 4 nitrogen or sulphur atoms, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- M represents a metal atom, optionally carrying from 1 to 2 substituents, R₆ and R₇, identical or different, which substituents are chosen from
• a halogen atom,
• a thio-osidic group with 3 to 60 carbon atoms, optionally substituted by one or more groups, identical or different, chosen from the protective groups of the hydroxyl or amine groups, such as an alkanoyl group with 2 to 6 carbon atoms, or an arylcarbonyl group with 6 to 14 carbon atoms, or
• a peptide group comprising from 1 to 5 amino acids, at least one of the amino acids comprising a thio group, chosen from S-cysteine or S-glutathione, or
• a linear or branched thioalkyl group with 1 to 6 carbon atoms, a saturated or unsaturated thiocycloalkyl group with 3 to 7 carbon atoms or a thioaryl or thioheteroaryl group with 6 to 14 carbon atoms, optionally substituted by one or more groups, identical or different, chosen from a halogen atom, an alkyl group with 1 to 6 carbon atoms, optionally substituted by one or more halogen atoms, or an aryl group with 6 to 14 carbon atoms;
in combination with a pharmaceutically acceptable vehicle.

3. A drug according to claim 1 or 2, **characterized in that** it comprises as active ingredient at least one compound of general formula (II) below: 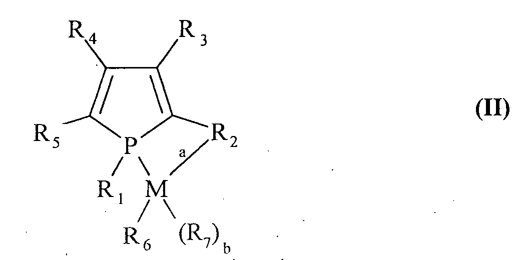 in which:
- a represents a single bond or no bond,
- b represents 0 or 1,
- R₁, R₂, R₃, R₄ and R₅ are as defined in claim 1 or 2,
- R₆ and R₇ represent, independently of one another,
• a halogen atom,
• a thio-osidic group with 3 to 60 carbon atoms, optionally substituted by one or more groups, identical or different, chosen from the protective groups of the hydroxyl or amine groups, such as an alkanoyl group with 2 to 6 carbon atoms, or an arylcarbonyl group with 6 to 14 carbon atoms, or
• a peptide group comprising from 1 to 5 amino acids, at least one of the amino acids comprising a thio group, chosen from S-cysteine or S-glutathione, or
• a linear or branched thioalkyl group with 1 to 6 carbon atoms, a saturated or unsaturated thiocycloalkyl group with 3 to 7 carbon atoms or a thioaryl or thioheteroaryl group with 6 to 14 carbon atoms, optionally substituted by one or more groups, identical or different, chosen from a halogen atom, an alkyl group with 1 to 6 carbon atoms, optionally substituted by one or more halogen atoms, or an aryl group with 6 to 14 carbon atoms;
- M represents a divalent, trivalent or tetravalent metal atom, providing that when M represents a divalent metal atom then a represents no bond and b is 0, when M represents a trivalent metal atom then a represents no bond and b is 1 and that when M represents a tetravalent metal atom then a represents a single bond and b is 1.

4. The drug according to one of claims 1 to 3, **characterized in that** it comprises as active ingredient at least one compound of general formula (III): 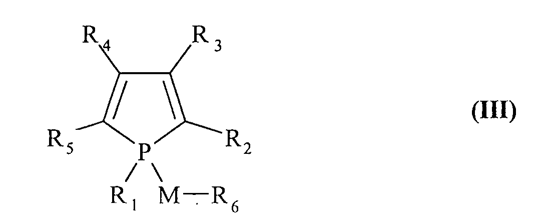 in which R₁, R₂, R₃, R₄, R₅ and R₆ are as defined in claim 1, 2 or 3, and M represents a divalent metal atom, such as Au.

5. The drug according to one of claims 1 to 4, **characterized in that** it comprises as active ingredient at least one compound of general formula (IV) below: 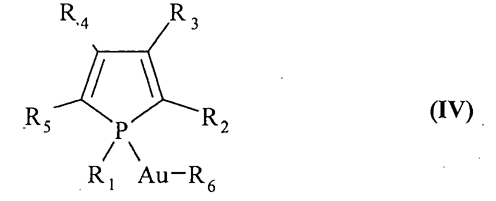 in which R₂, R₃, R₄, R₅ and R₆ are as defined in claim 1, 2 or 3.

6. The drug according to one of claims 1 to 5, **characterized in that** it comprises as active ingredient at least one compound of general formula (IV') below: 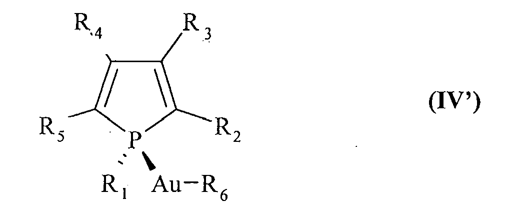 in which R₂, R₃, R₄, R₅ and R₆ are as defined in claim 1, 2 or 3.

7. The drug according to one of claims 1 to 3, **characterized in that** it comprises as active ingredient at least one compound of general formula (V): 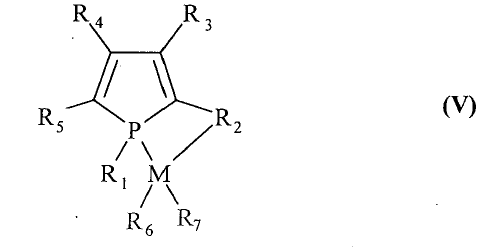 in which R₁, R₂, R₃, R₄, R₅, R₆ and R₇ are as defined in claim 1, 2 or 3, and M represents a tetravalent metal atom, such as Pt.

8. The drug according to one of claims 1 to 3 and 7, **characterized in that** it comprises as active ingredient at least one compound of general formula (VI) below: 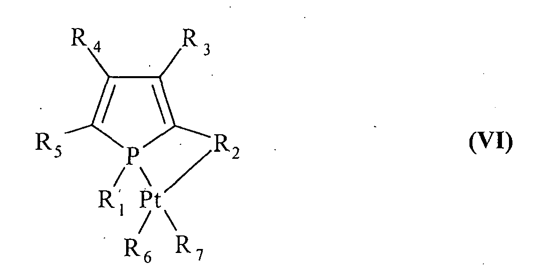 in which R₂, R₃, R₄, R₅ and R₆ are as defined in claim 1, 2 or 3.

9. The drug according to one of claims 1 to 3, 7 and 8, **characterized in that** it comprises as active ingredient at least one compound of general formula (VI') below: 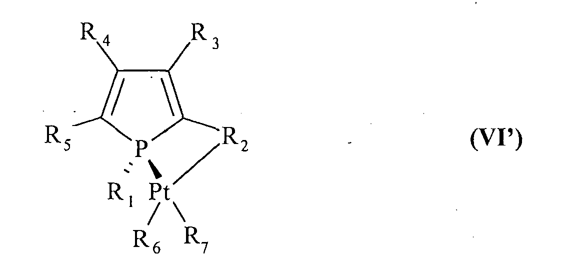 in which R₂, R₃, R₄, R₅ and R₆ are as defined in claim 1, 2 or 3.

10. The drug according to one of claims 1 to 3 and 7 to 9, **characterized in that** it comprises as active ingredient at least one compound of general formula (VII) below: 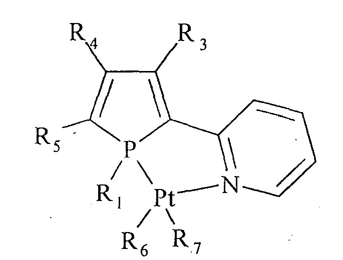 in which R₁, R₃, R₄, R₅, R₆ and R₇ are as defined in claim 1, 2 or 3.

11. The drug according to one of claims 1 to 3 and 7 to 10, **characterized in that** it comprises as active ingredient at least one compound of general formula (VII') below: 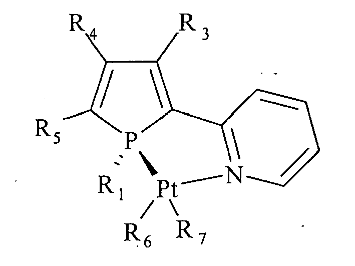 in which R₁, R₃, R₄, R₅, R₆ and R₇ are as defined in claim 1, 2 or 3.

12. The drug according to one of claims 1 to 11, **characterized in that:**
- R₁ represents a cycloalkyl group with 3 to 7 carbon atoms or an aryl group with 6 to 14 carbon atoms, (and more particularly a cyclohexyl or phenyl group);
- R₂ represents a nitrogen-containing heteroaryl group with six atoms having from 1 to 3 nitrogen atoms, (and more particularly the 2-pyridyl group);
- R₃ and R₄ represent independently of one another an alkyl group with 1 to 6 carbon atoms, and (more particularly) an alkyl group with 3 or 4 carbon atoms linking the atoms in positions 3 and 4 of the phosphole ring;
- R₅ represents an aryl group with 6 to 14 carbon atoms, a thienyl group, or a pyridyl group, substituted or non-substituted, (and more particularly a phenyl, 2-thienyl or 2-pyridyl group);
- R₆ and optionally R₇ represent independently of one another a halogen, (and more particularly a chlorine atom), a thio-monosaccharide, (in particular) chosen from a thiopentose or a thiohexose, or a thiodioside, optionally substituted by one or more protective groups of the hydroxyl or amine groups, and more particularly thioglucose, thioglucose tetraacetate, thiomannose, thiomannose tetraacetate, thiogalactose, thiogalactose tetraacetate, thioribose, thioribose triacetate, thioxylose, thioxylose triacetate, thio-allose, thio-allose tetraacetate, thiotalose, thiotalose tetraacetate, thiofucose, thiofucose tetraacetate, thio-N-acetyl-glucosamine, thio-N-acetyl-glucosamine triacetate, thio-N-acetyl-galactosamine, thio-N-acetyl-galactosamine triacetate, thio-N-acetyl mannosamine, thio-N-acetyl mannosamine triacetate, thiolactose, thiolactose hepta-acetate.

13. The drug according to one of claims 1 to 12, **characterized in that** it comprises as active ingredient at least one compound of the following formula: 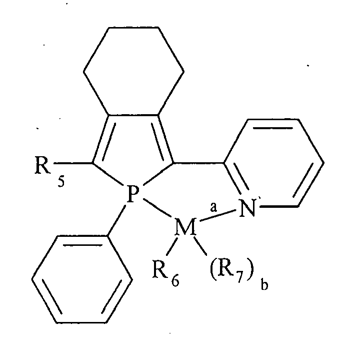 in which R₅ represents a 2-thienyl or 2-pyridyl group, M represents Au or Pt, a represents a single bond when M represents Pt or no bond when M represents Au, b represents 1 when M represents Pt and 0 when M represents Au, and R₆ and R₇ are as defined in claim 12.

14. The drug according to claim 13, **characterized in that** it comprises as active ingredient at least one compound of the following formula: 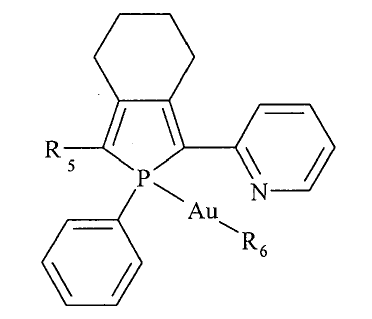 in which R₅ represents a 2-thienyl or 2-pyridyl group and R₆ is as defined in claim 12.

15. The drug according to one of claims 1 to 14, **characterized in that** it comprises as active ingredient at least one compound of the following formula:

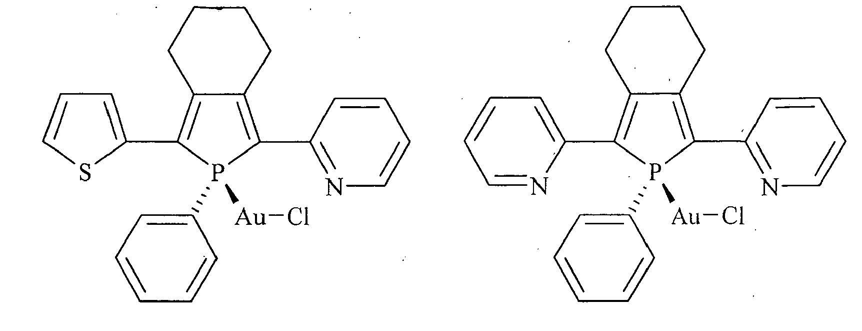

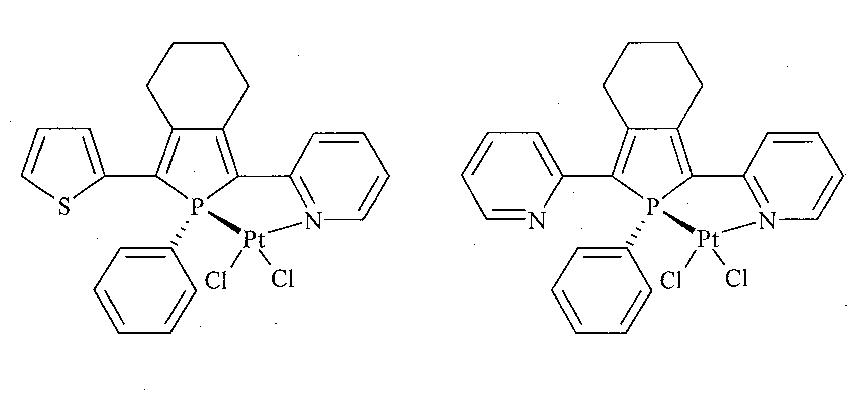

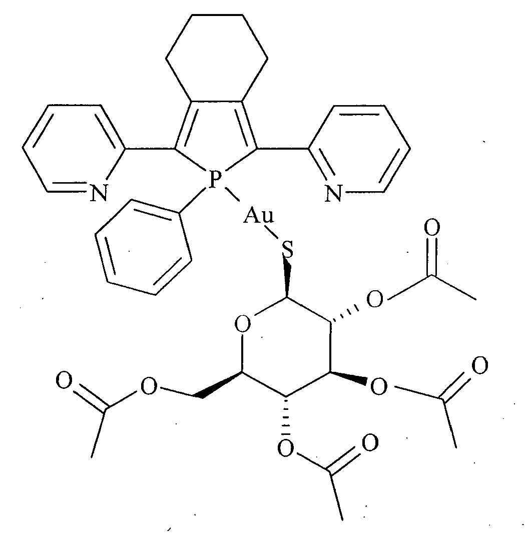

16. The drug according to claim 1, **characterized in that** it comprises as active ingredient at least one compound of general formula (VIII) below: 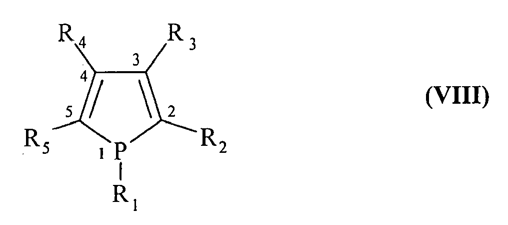 in which:
- R₁ represents a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, or an aryl group with 6 to 14 carbon atoms;
- R₂ represents a nitrogen-containing heteroaryl group or a nitrogen-containing heterocycle with 5 or 6 atoms having from 1 to 4 nitrogen atoms;
- R₃ and R₄ represent, independently of one another, a hydrogen atom, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, or an aryl group with 6 to 14 carbon atoms, optionally an alkyl group can bridge the carbon atoms in positions 3 and 4 of the phosphole ring carrying, respectively, the substituents R₃ and R₄, so as to form a ring with 3 to 8 carbon atoms;
- R₅ represents a hydrogen atom, a heterocycle with five or six members having from 1 to 4 nitrogen or sulphur atoms, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, or an aryl group with 6 to 14 carbon atoms;
in combination with a pharmaceutically acceptable vehicle.

17. The drug according to claim 16, **characterized in that:**
- R₁ represents a cycloalkyl group with 3 to 7 carbon atoms or an aryl group with 6 to 14 carbon atoms, and more particularly a cyclohexyl or phenyl group;
- R₂ represents a nitrogen-containing heteroaryl group with six atoms having from 1 to 3 nitrogen atoms, and more particularly the 2-pyridyl group;
- R₃ and R₄ represent independently of one another an alkyl group with 1 to 6 carbon atoms, and (more particularly) an alkyl group with 3 or 4 carbon atoms linking the atoms in positions 3 and 4 of the phosphole ring;
- R₅ represents an aryl group with 6 to 14 carbon atoms, a thienyl group, or a pyridyl group, substituted or non-substituted, and more particularly a phenyl, 2-thienyl or 2-pyridyl group.

18. The drug according to claim 16 or 17, it comprises as active ingredient at least one compound of the following formula: 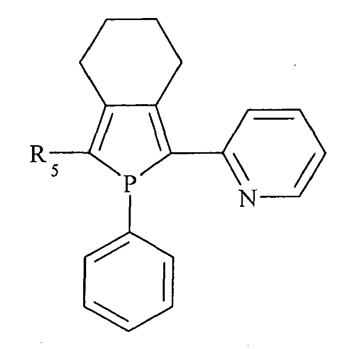 in which R₅ is as defined in claim 17.

19. The drug according to one of claims 16 to 18, **characterized in that** it comprises as active ingredient at least one compound of the following formula:

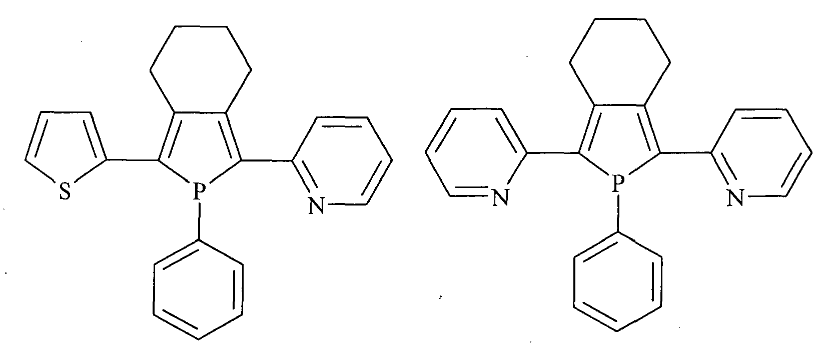

20. The drug according to one of claims 1 to 19, **characterized in that** it comprises as active ingredient at least one compound of general formula (A), and more particularly of formula (I) to (VIII), in combination with at least one anticancer compound such as cisplatin, mitomycin C, doxorubicin, etoposide, carmustine.

21. Products comprising:
* at least one compound of general formula (A), and more particularly of formula (I) to (VIII), as defined in one of claims 1 to 19,
* and at least one anticancer compound, as defined in claim 20,
as combination products for simultaneous or separate use, or use spread over time, in cancer therapy.

22. The combination product according to claim 21, **characterized in that** it contains a product of formula (A) and an anticancer agent in a ratio of approximately 0.1/1 to approximately 2.5/1 and, optionally, one or more pharmaceutically acceptable vehicles.

23. The drugs or combination products, according to one of claims 1 to 22, **characterized in that** they are present in a form which can be administered by intravenous route.

24. The drugs or combination products, according to one of claims 1 to 23, **characterized in that** the dosage of the compounds of formula (A) contained in these pharmaceutical compositions or combination products, is approximately 5 to approximately 200 mg /m²/day or per cure.

25. Use of at least one compound of general formula (I) as defined in one of claims 2 to 15 for the preparation of a medicament intended for the prevention or treatment of pathologies linked to excessive glutathione reductase and/or thioredoxin reductase activity.

26. Use according to claim 25, **characterized in that** the pathologies linked to excessive glutathione reductase and/or thioredoxin reductase activity, are:
- cancers, namely all liquid or solid tumours, and more particularly cancers associated with infection by the Epstein-Barr virus,
- rheumatoid arthritis,
- psoriasis or psoriatic rheumatism,
- Sjögren's syndrome,
- infection with the HIV virus and associated diseases such as Kaposi's carcinoma.

27. Use of at least one compound of formula (VIII) as defined in one of claims 16 to 19 for the preparation of a medicament intended for the treatment of cancers.

28. Use of at least one compound of general formula (A) as defined in one of claims 1 to 19 for the preparation of a medicament intended for the prevention or treatment of the phenomena of resistance to anticancer medicaments such as cisplatin, mitomycin C, doxorubicin, etoposide, carmustine, and, optionally anti-infectious medicaments such as metronidazole, isoniazid.

29. **A** compound of general formula (IX) below: 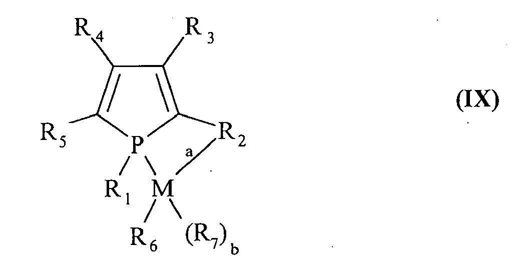 in which:
- a represents a single bond or no bond,
- b represents 0 or 1,
- R₁ represents a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- R₂ represents a nitrogen-containing heteroaryl group or a nitrogen-containing heterocycle with 5 or 6 atoms having from 1 to 4 nitrogen atoms;
- R₃ and R₄ represent, independently of one another, a hydrogen atom, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms, optionally an alkyl group can bridge the carbon atoms in positions 3 and 4 of the phosphole ring carrying, respectively, the substituents R₃ and R₄, so as to form a ring with 3 to 8 carbon atoms;
- R₅ represents a hydrogen atom, a heterocycle with five or six members having from 1 to 4 nitrogen or sulphur atoms, or a
• linear or branched alkyl group with 1 to 6 carbon atoms,
• saturated or unsaturated cycloalkyl group with 3 to 7 carbon atoms,
• aryl group with 6 to 14 carbon atoms,
• linear or branched alkoxy group with 1 to 6 carbon atoms,
• saturated or unsaturated cycloalkoxy group with 3 to 7 carbon atoms,
• aryloxy group with 6 to 14 carbon atoms,
• linear or branched alkylthio group with 1 to 6 carbon atoms,
• saturated or unsaturated cycloalkylthio group with 3 to 7 carbon atoms, or
• arylthio group with 6 to 14 carbon atoms,
optionally substituted by one or more groups, identical or different, chosen from a halogen atom, a linear or branched alkyl group with 1 to 6 carbon atoms, optionally substituted by one or more halogen atoms, or an aryl group with 6 to 14 carbon atoms;
- M represents a divalent, trivalent or tetravalent metal atom, providing that when M represents a divalent metal atom then a represents no bond and b is 0, when M represents a trivalent metal atom then a represents no bond and b is 1 and that when M represents a tetravalent metal atom then a represents a single bond and b is 1,
- R₆ represents a thio-osidic group with 3 to 60 carbon atoms, optionally substituted by one or more groups, identical or different, chosen from the protective groups of the hydroxyl or amine groups, such as an alkanoyl group with 2 to 6 carbon atoms, or an arylcarbonyl group with 6 to 14 carbon atoms,
- R₇ represents
• a halogen atom,
• a thio-osidic group with 3 to 60 carbon atoms, optionally substituted by one or more groups, identical or different, chosen from the protective groups of the hydroxyl or amine groups, such as an alkanoyl group with 2 to 6 carbon atoms, or an arylcarbonyl group with 6 to 14 carbon atoms,
• a peptide group comprising from 1 to 5 amino acids, at least one of the amino acids comprising a thio group, such as S-cysteine or S-glutathione, or
• a linear or branched thioalkyl group with 1 to 6 carbon atoms, a saturated or unsaturated thiocycloalkyl group with 3 to 7 carbon atoms or a thioaryl or thioheteroaryl group with 6 to 14 carbon atoms, optionally substituted by one or more groups, identical or different, chosen from a halogen atom, an alkyl group with 1 to 6 carbon atoms, optionally substituted by one or more halogen atoms, or an aryl group with 6 to 14 carbon atoms.

30. The compound according to claim 29, of general formula (X): 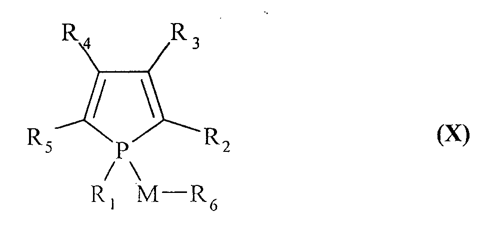 in which R₁, R₂, R₃, R₄, R₅ and R₆ are as defined in claim 29, and M represents a divalent metal atom, such as Au.

31. The compound according to claim 29 or 30, of general formula (XI): 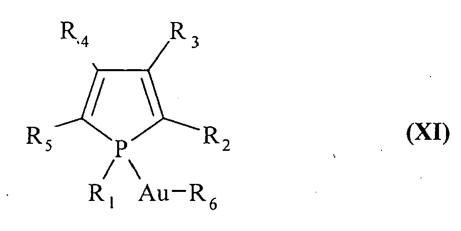 in which R₂, R₃, R₄, R₅ and R₆ are as defined in claim 26.

32. The compound according to one of claims 29 to 31, in which:
- R₁ represents a cycloalkyl group with 3 to 7 carbon atoms or an aryl group with 6 to 14 carbon atoms, and more particularly a cyclohexyl or phenyl group;
- R₂ represents a nitrogen-containing heteroaryl group with six atoms having from 1 to 3 nitrogen atoms, and more particularly the 2-pyridyl group;
- R₃ and R₄ represent independently of one another an alkyl group with 1 to 6 carbon atoms, and more particularly an alkyl group with 3 or 4 carbon atoms linking the atoms in positions 3 and 4 of the phosphole ring;
- R₅ represents an aryl group with 6 to 14 carbon atoms, a thienyl group, or a pyridyl group, substituted or non-substituted, (and more particularly a phenyl, 2-thienyl or 2-pyridyl group);
- R₆ represents a thio-monosaccharide, in particular chosen from a thiopentose or a thiohexose, or a thiodioside, optionally substituted by one or more protective groups of the hydroxyl or amine groups, and more particularly thioglucose, thioglucose tetraacetate, thiomannose, thiomannose tetraacetate, thiogalactose, thiogalactose tetraacetate, thioribose, thioribose triacetate, thioxylose, thioxylose triacetate, thio-allose, thio-allose tetraacetate, thiotalose, thiotalose tetraacetate, thiofucose, thiofucose tetraacetate, thio-N-acetyl-glucosamine, thio-N-acetyl-glucosamine triacetate, thio-N-acetyl-galactosamine, thio-N-acetyl-galactosamine triacetate, thio-N-acetyl mannosamine, thio-N-acetyl mannosamine triacetate, thiolactose, thiolactose hepta-acetate;
- optionally, R₇ represents a halogen, and more particularly a chlorine atom, a thio-monosaccharide, in particular chosen from a thiopentose or a thiohexose, or a thiodioside, optionally substituted by one or more protective groups of the hydroxyl or amine groups, and more particularly thioglucose, thioglucose tetraacetate, thiomannose, thiomannose tetraacetate, thiogalactose, thiogalactose tetraacetate, thioribose, thioribose triacetate, thioxylose, thioxylose triacetate, thio-allose, thio-allose tetraacetate, thiotalose, thiotalose tetraacetate, thiofucose, thiofucose tetraacetate, thio-N-acetyl-glucosamine, thio-N-acetyl-glucosamine triacetate, thio-N-acetyl-galactosamine, thio-N-acetyl-galactosamine triacetate, thio-N-acetyl mannosamine, thio-N-acetyl mannosamine triacetate, thiolactose, thiolactose hepta-acetate.

33. The compound according to one of claims 29 to 32, of the following formula: 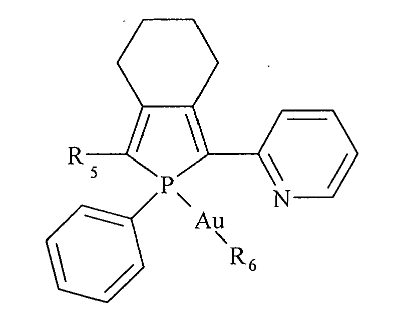 in which R₅ and R₆ are as defined in claim 32.

34. The compound according to one of claims 29 to 33, of the following formula:

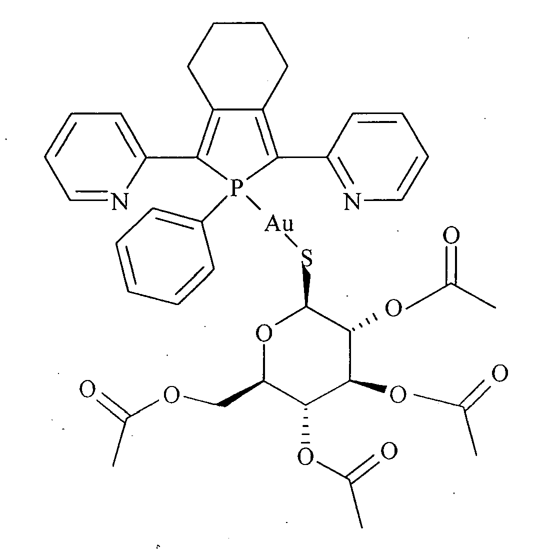

## Patentansprüche

1. Medikament, **dadurch gekennzeichnet, dass** es als Wirkstoff mindestens eine Verbindung der folgenden allgemeinen Formel (A) umfasst: 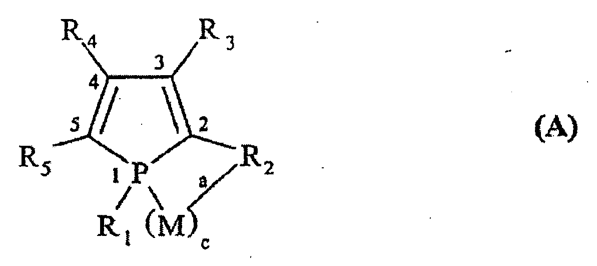 worin:
- a für eine Einfachbindung oder keine Bindung steht,
- c für 0 steht, wenn M abwesend ist, oder 1, wenn M vorhanden ist,
- R₁ für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen,
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- R₂ für eine Stickstoff enthaltende Heteroarylgruppe oder einen Stickstoff enthaltenden Heteroring mit 5 oder 6 Atomen steht, der 1 bis 4 Stickstoffatome besitzt;
- R₃ und R₄ jeweils unabhängig voneinander für ein Wasserstoffatom oder eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen,
• eine Arylthiogruppe mit 6 bis 14 Kohlenstoffatomen stehen,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen, wobei gegebenenfalls eine Alkylgruppe die Kohlenstoffatome an den Positionen 3 und 4 des Phospholrings untereinander verbinden kann, die jeweils die Substituenten R₃ und R₄ tragen, um einen Ring mit 3 bis 8 Kohlenstoffatomen zu bilden;
- R₅ für ein Wasserstoffatom, einen Heteroring mit fünf oder sechs Gliedern steht, der 1 bis 4 Stickstoff- oder Schwefelatome besitzt, oder für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen,
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- wobei gegebenenfalls (b = 1) M für ein Metallatom steht, das optional 1 bis 2 Substituenten trägt, R₆ und R₇, gleich oder verschieden, wobei die Substituenten ausgewählt sind aus
• einem Halogenatom,
• einer Thioglycosidgruppe mit 3 bis 60 Kohlenstoffatomen, die optional mit einer oder mehreren Gruppen, gleich oder verschieden, substituiert ist, ausgewählt aus den Schutzgruppen der Hydroxyl- oder Amingruppen, wie etwa einer Alkanoylgruppe mit 2 bis 6 Kohlenstoffatomen, oder einer Arylcarbonylgruppe mit 6 bis 14 Kohlenstoffatomen,
• einer Peptidgruppe, die 1 bis 5 Aminosäuren umfasst, wobei mindestens eine der Aminosäuren eine Thiogruppe umfasst, wie etwa S-Cystein oder S-Glutathion, oder
• einer linearen oder verzweigten Thioalkylgruppe mit 1 bis 6 Kohlenstoffatomen, einer gesättigten oder ungesättigten Thiocycloalkylgruppe mit 3 bis 7 Kohlenstoffatomen oder einer Thioaryl- oder Thioheteroarylgruppe mit 6 bis 14 Kohlenstoffatomen, die optional mit einer oder mehreren Gruppen, gleich oder verschieden, substituiert ist, ausgewählt aus einem Halogenatom, einer Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
in Kombination mit einem pharmazeutisch unbedenklichen Vehikel.

2. Medikament nach Anspruch 1, **dadurch gekennzeichnet, dass** es als Wirkstoff mindestens eine Verbindung der folgenden allgemeinen Formel (I) umfasst: 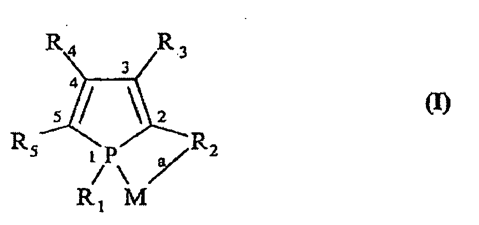 worin:
- a für eine Einfachbindung oder keine Bindung steht,
- R₁ für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- R₂ für eine Stickstoff enthaltende Heteroarylgruppe oder einen Stickstoff enthaltenden Heteroring mit 5 oder 6 Atomen steht, der 1 bis 4 Stickstoffatome besitzt;
- R₃ und R₄ jeweils unabhängig voneinander für ein Wasserstoffatom oder eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen, oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen, wobei gegebenenfalls eine Alkylgruppe die Kohlenstoffatome an den Positionen 3 und 4 des Phospholrings untereinander verbinden kann, die jeweils die Substituenten R₃ und R₄ tragen, um einen Ring mit 3 bis 8 Kohlenstoffatomen zu bilden;
- R₅ für ein Wasserstoffatom, einen Heteroring mit fünf oder sechs Gliedern steht, der 1 bis 4 Stickstoff- oder Schwefelatome besitzt, oder für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- M für ein Metallatom steht, das optional 1 bis 2 Substituenten, R₆ und R₇, gleich oder verschieden, trägt, wobei die Substituenten ausgewählt sind aus
• einem Halogenatom,
• einer Thioglycosidgruppe mit 3 bis 60 Kohlenstoffatomen, die optional mit einer oder mehreren Gruppen, gleich oder verschieden, substituiert ist, ausgewählt aus den Schutzgruppen der Hydroxyl- oder Amingruppen, wie etwa einer Alkanoylgruppe mit 2 bis 6 Kohlenstoffatomen, oder einer Arylcarbonylgruppe mit 6 bis 14 Kohlenstoffatomen oder
• einer Peptidgruppe, die 1 bis 5 Aminosäuren umfasst, wobei mindestens eine der Aminosäuren eine Thiogruppe umfasst, wie etwa S-Cystein oder S-Glutathion, oder
• einer linearen oder verzweigten Thioalkylgruppe mit 1 bis 6 Kohlenstoffatomen, einer gesättigten oder ungesättigten Thiocycloalkylgruppe mit 3 bis 7 Kohlenstoffatomen oder einer Thioaryl- oder Thioheteroarylgruppe mit 6 bis 14 Kohlenstoffatomen, die optional mit einer oder mehreren Gruppen, gleich oder verschieden, substituiert ist, ausgewählt aus einem Halogenatom, einer Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
in Kombination mit einem pharmazeutisch unbedenklichen Vehikel.

3. Medikament nach Anspruch 1 oder 2, **dadurch gekennzeichnet, dass** es als Wirkstoff mindestens eine Verbindung der folgenden allgemeinen Formel (II) umfasst: 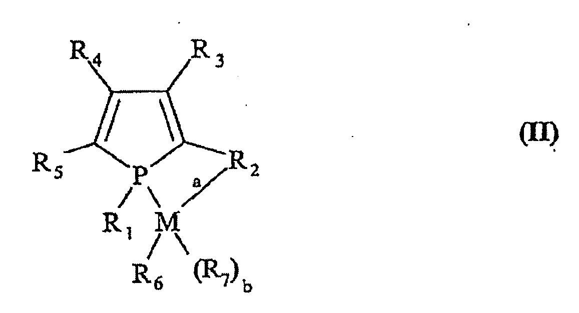 worin:
- a für eine Einfachbindung oder keine Bindung steht,
- b für 0 oder 1 steht,
- R₁, R₂, R₃, R₄ und R₅ sind, wie in Anspruch 1 oder 2 definiert,
- R₆ und R₇ unabhängig voneinander für
• ein Halogenatom,
• eine Thioglycosidgruppe mit 3 bis 60 Kohlenstoffatomen, die optional mit einer oder mehreren Gruppen, gleich oder verschieden, substituiert ist, ausgewählt aus den Schutzgruppen der Hydroxyl- oder Amingruppen, wie etwa einer Alkanoylgruppe mit 2 bis 6 Kohlenstoffatomen, oder einer Arylcarbonylgruppe mit 6 bis 14 Kohlenstoffatomen,
• eine Peptidgruppe, die 1 bis 5 Aminosäuren umfasst, wobei mindestens eine der Aminosäuren eine Thiogruppe umfasst, wie etwa S-Cystein oder S-Glutathion, oder
• eine lineare oder verzweigte Thioalkylgruppe mit 1 bis 6 Kohlenstoffatomen, eine gesättigte oder ungesättigte Thiocycloalkylgruppe mit 3 bis 7 Kohlenstoffatomen oder eine Thioaryl- oder Thioheteroarylgruppe mit 6 bis 14 Kohlenstoffatomen, die optional mit einer oder mehreren Gruppen, gleich oder verschieden, substituiert ist, ausgewählt aus einem Halogenatom, einer Alkylgruppe mit 1 bis 6 Kohlenstoffatomen, die optional mit einem oder mehreren Halogenatomen substituiert ist, oder eine Arylgruppe mit 6 bis 14 Kohlenstoffatomen stehen;
- M für ein bivalentes, trivalentes oder tetravalentes Metallatom steht, unter der Bedingung, dass, wenn M für ein bivalentes Metallatom steht, a für keine Bindung steht und b gleich 0 ist, wenn M für ein trivalentes Metallatom steht, a für keine Bindung steht und b gleich 1 ist, und wenn M für eine tetravalentes Metallatom steht, a für eine Einfachbindung steht und b gleich 1 ist.

4. Medikament nach einem der Ansprüche 1 bis 3, **dadurch gekennzeichnet, dass** es als Wirkstoff mindestens eine Verbindung der allgemeinen Formel (III) umfasst: 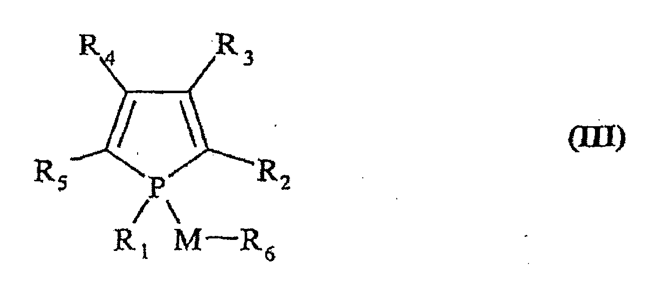 worin R₁, R₂, R₃, R₄, R₅ und R₆ sind, wie in Anspruch 1, 2 oder 3 definiert, und M für ein bivalentes Metallatom, wie etwa Au, steht.

5. Medikament nach einem der Ansprüche 1 bis 4, **dadurch gekennzeichnet, dass** es als Wirkstoff mindestens eine Verbindung der folgenden allgemeinen Formel (IV) umfasst: 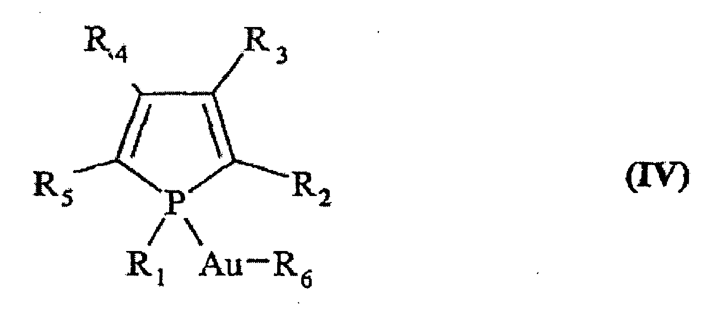 worin R₂, R₃, R₉, R₅ und R₆ sind, wie in Anspruch 1, 2 oder 3 definiert,

6. Medikament nach einem der Ansprüche 1 bis 5, **dadurch gekennzeichnet, dass** es als Wirkstoff mindestens eine Verbindung der folgenden allgemeinen Formel (IV') umfasst: 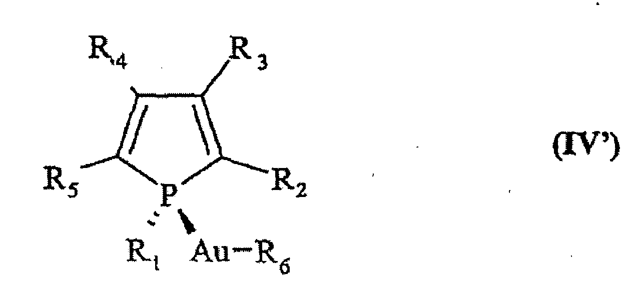
- worin R₂, R₃, R₄, R₅ und R₆ sind, wie in Anspruch 1, 2 oder 3 definiert,

7. Medikament nach einem der Ansprüche 1 bis 3, **dadurch gekennzeichnet, dass** es als Wirkstoff mindestens eine Verbindung der allgemeinen Formel (III) umfasst: 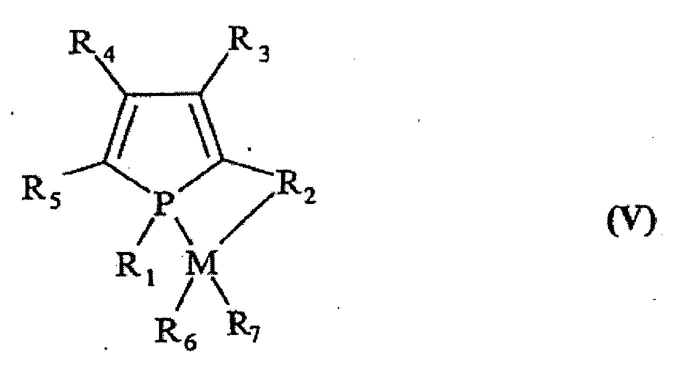 worin R₁, R₂, R₃, P₄, R₅, R₆ und R₇ sind, wie in Anspruch 1, 2 oder 3 definiert, und M für ein tetravalentes Metallatom, wie etwa Pt, steht.

8. Medikament nach einem der Ansprüche 1 bis 3 und 7, **dadurch gekennzeichnet, dass** es als Wirkstoff mindestens eine Verbindung der folgenden allgemeinen Formel (VI) umfasst: 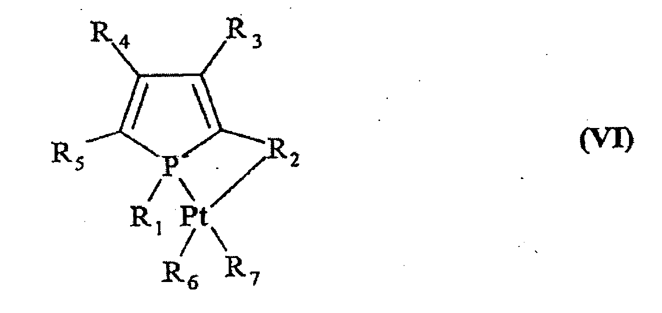 worin R₂, R₃, R₄, R₅ und R₆ sind, wie in Anspruch 1, 2 oder 3 definiert,

9. Medikament nach einem der Ansprüche 1 bis 3, 7 und 8, **dadurch gekennzeichnet, dass** es als Wirkstoff mindestens eine Verbindung der folgenden allgemeinen Formel (VI') umfasst: 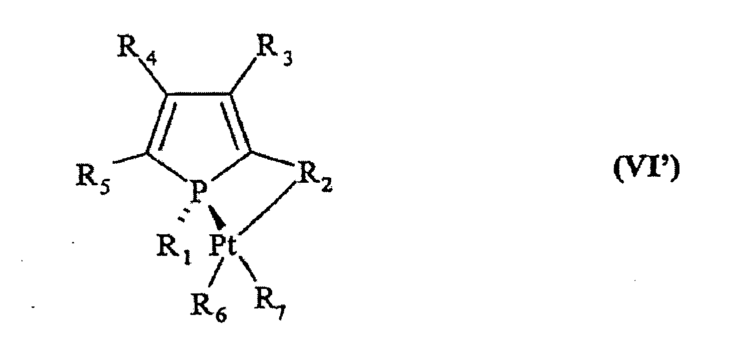 worin R₂, R₃, R₄, R₅ und R₆ sind, wie in Anspruch 1, 2 oder 3 definiert,

10. Medikament nach einem der Ansprüche 1 bis 3 und 7 bis 9, **dadurch gekennzeichnet, dass** es als Wirkstoff mindestens eine Verbindung der folgenden allgemeinen Formel (VII) umfasst: 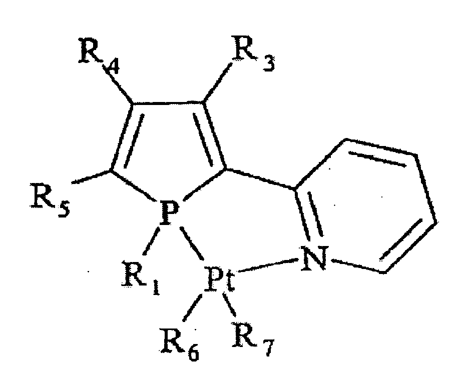 worin R₁, R₃, R₄, R₅, R₆ und R₇ sind, wie in Anspruch 1, 2 oder 3 definiert,

11. Medikament nach einem der Ansprüche 1 bis 3 und 7 bis 10, **dadurch gekennzeichnet, dass** es als Wirkstoff mindestens eine Verbindung der folgenden allgemeinen Formel (VII') umfasst: 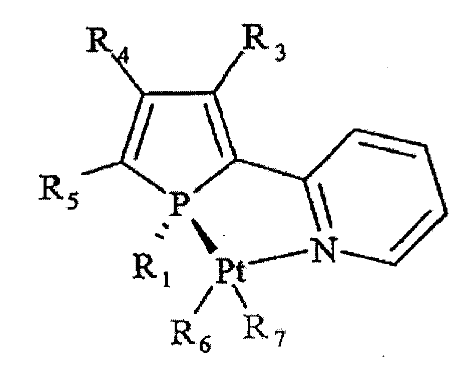
- worin R₁, R₃, R₄, R₅, R₆ und R₇ sind, wie in Anspruch 1, 2 oder 3 definiert,

12. Medikament nach einem der Ansprüche 1 bis 11, **dadurch gekennzeichnet, dass:**
- R₁ für eine Cycloalkylgruppe mit 3 bis 7 Kohlenstoffatomen oder eine Arylgruppe mit 6 bis 14 Kohlenstoffatomen, und insbesondere für eine Cyclohexyl- oder Phenylgruppe steht;
- R₂ für eine Stickstoff enthaltende Heteroarylgruppe mit sechs Atomen, die 1 bis 3 Stickstoffatome besitzt, und insbesondere für die 2-Pyridylgruppe steht;
- R₃ und R₄ unabhängig voneinander für Alkylgruppen mit 1 bis 6 Kohlenstoffatomen und insbesondere für eine Akylgruppe mit 3 oder 4 Kohlenstoffatomen stehen, die die Atome an den Positionen 3 und 4 des Phospholrings verbindet;
- R₅ für eine Arylgruppe mit 6 bis 14 Kohlenstoffatomen, eine substituierte oder nicht substituierte Thienyl- oder Pyridylgruppe, und insbesondere für eine Phenyl-, 2-Thienyl- oder 2-Pyridylgruppe steht;
- R₆ und gegebenenfalls R₇ unabhängig voneinander für ein Halogen, und insbesondere ein Chloratom, ein Thiomonosacharid, vor allem ausgewählt aus Thiopentose oder Thiohexose, oder ein Thiodiosid, das optional mit einer oder mehreren Schutzgruppen der Hydroxyl- oder Amingruppen substituiert ist, und insbesondere Thioglucose, Thioglucosetetraacetat, Thiomannose, Thiomannosetetraacetat, Thiogalactose, Thiogalactosetetraacetat, Thioribose, Thioribosetriacetat, Thioxylose, Thioxylosetriacetat, Thioallose, Thioallosetetraacetat, Thiotalose, Thiotalosetetraacetat, Thiofucose, Thiofucosetetraacetat, Thio-N-acetyl-glucosamin, Thio-N-acetyl-glucosamintriacetat, Thio-N-acetylgalactosamin, Thio-N-acetylgalactosamintriacetat, Thio-N-acetylmannosamin, Thio-N-acetylmannosamintriacetat, Thiolactose, Thiolactoseheptaacetat stehen.

13. Medikament nach einem der Ansprüche 1 bis 12, **dadurch gekennzeichnet, dass** es als Wirkstoff mindestens eine Verbindung der folgenden Formel umfasst: 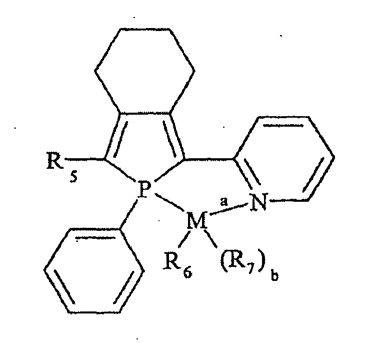 worin R₅ für eine 2-Thienyl- oder 2-Pyridylgruppe steht, M für Au oder Pt steht, a für eine Einfachbindung steht, wenn M für Pt steht, oder für keine Bindung, wenn M für Au steht, b für 1 steht, wenn M für Pt steht und für 0, wenn M für Au steht, und R₆ und R₇ sind, wie in Anspruch 12 definiert.

14. Medikament nach Anspruch 13, **dadurch gekennzeichnet, dass** es als Wirkstoff mindestens eine Verbindung der folgenden Formel umfasst: 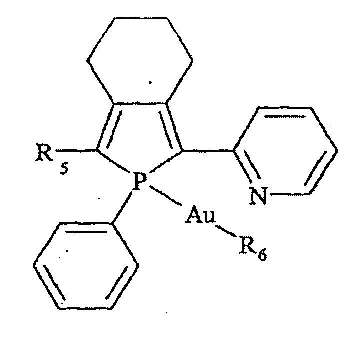 worin R₅ für eine 2-Thienyl- oder 2-Pyridylgruppe steht und R₆ ist, wie in Anspruch 12 definiert.

15. Medikament nach einem der Ansprüche 1 bis 14, **dadurch gekennzeichnet, dass** es als Wirkstoff mindestens eine Verbindung der folgenden Formel umfasst:

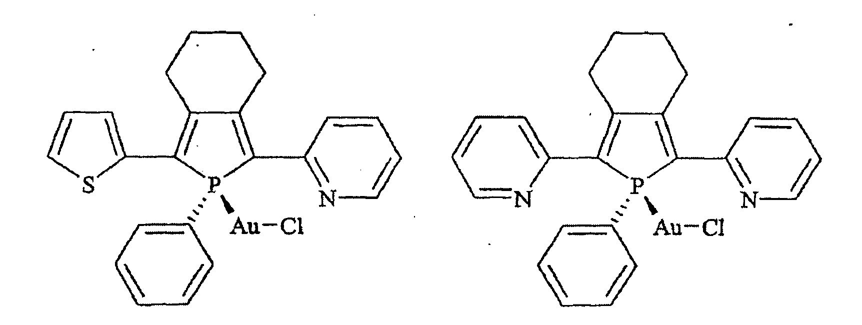

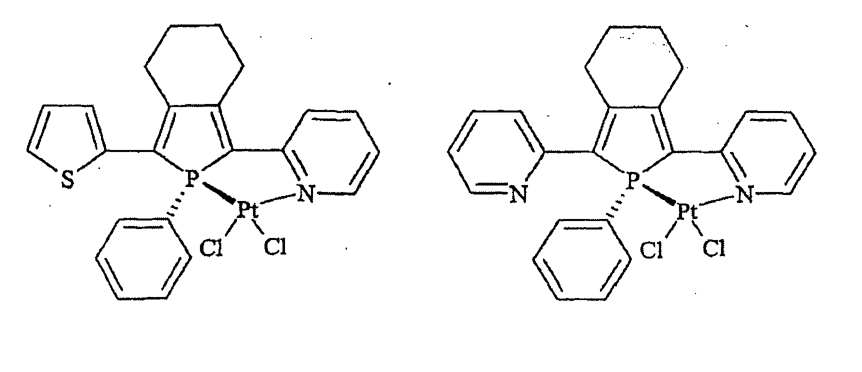

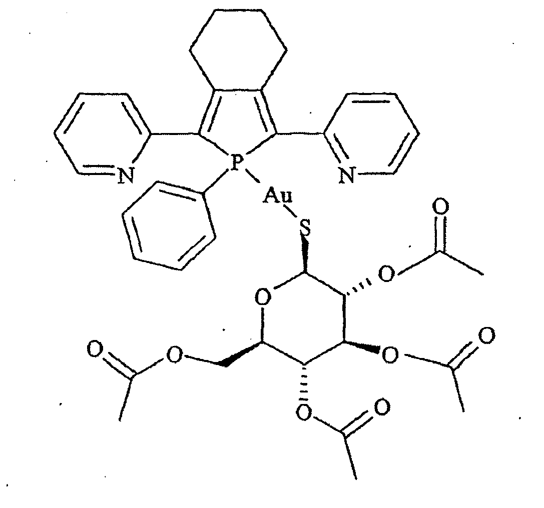

16. Medikament nach Anspruch 1, **dadurch gekennzeichnet, dass** es als Wirkstoff mindestens eine Verbindung der folgenden allgemeinen Formel (VIII) umfasst: 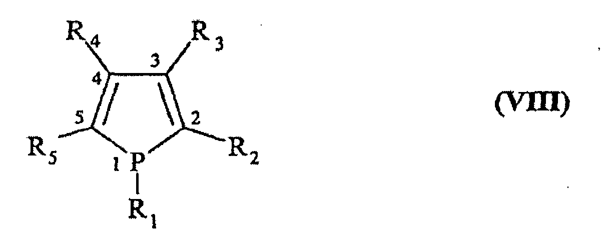 worin:
- R₁ für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, oder einer Arylgruppe mit 6 bis 14 Kohlenstoffatomen;
- R₂ für eine Stickstoff enthaltende Heteroarylgruppe oder einen Stickstoff enthaltenden Heteroring mit 5 oder 6 Atomen steht, der 1 bis 4 Stickstoffatome besitzt;
- R₃ und R₄ jeweils unabhängig voneinander für ein Wasserstoffatom oder eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen stehen,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, oder einer Arylgruppe mit 6 bis 14 Kohlenstoffatomen, wobei gegebenenfalls eine Alkylgruppe die Kohlenstoffatome an den Positionen 3 und 4 des Phospholrings untereinander verbinden kann, die jeweils die Substituenten R₃ und R₄ tragen, um einen Ring mit 3 bis 8 Kohlenstoffatomen zu bilden;
- R₅ für ein Wasserstoffatom, einen Heteroring mit fünf oder sechs Gliedern steht, der 1 bis 4 Stickstoff- oder Schwefelatome besitzt, oder für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, oder einer Arylgruppe mit 6 bis 14 Kohlenstoffatomen;
in Kombination mit einem pharmazeutisch unbedenklichen Vehikel.

17. Medikament nach Anspruch 16, **dadurch gekennzeichnet, dass:**
- R₁ für eine Cycloalkylgruppe mit 3 bis 7 Kohlenstoffatomen oder eine Arylgruppe mit 6 bis 14 Kohlenstoffatomen, und insbesondere für eine Cyclohexyl- oder Phenylgruppe steht;
- R₂ für eine Stickstoff enthaltende Heteroarylgruppe mit sechs Atomen, die 1 bi 3 Stickstoffatome besitzt, und insbesondere für die 2-Pyridylgruppe steht;
- R₃ und R₄ unabhängig voneinander für Alkylgruppen mit 1 bis 6 Kohlenstoffatomen und insbesondere für eine Akylgruppe mit 3 oder 4 Kohlenstoffatomen stehen, die die Atome an den Positionen 3 und 4 des Phospholrings verbinden;
- R₅ für eine Arylgruppe mit 6 bis 14 Kohlenstoffatomen, eine substituierte oder nicht substituierte Thienyl- oder Pyridylgruppe, und insbesondere für eine Phenyl-, 2-Thienyl- oder 2-Pyridylgruppe steht.

18. Medikament nach Anspruch 16 oder 17, **dadurch gekennzeichnet, dass** es als Wirkstoff mindestens eine Verbindung der folgenden Formel umfasst: 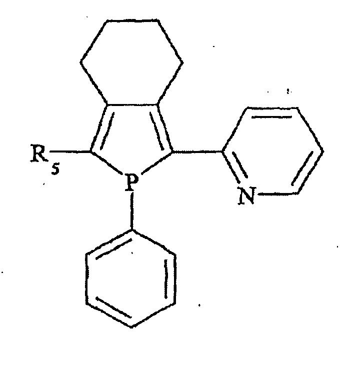 worin R₅ ist, wie in Anspruch 17 definiert.

19. Medikament nach einem der Ansprüche 16 bis 18, **dadurch gekennzeichnet, dass** es als Wirkstoff mindestens eine Verbindung der folgenden Formel umfasst:

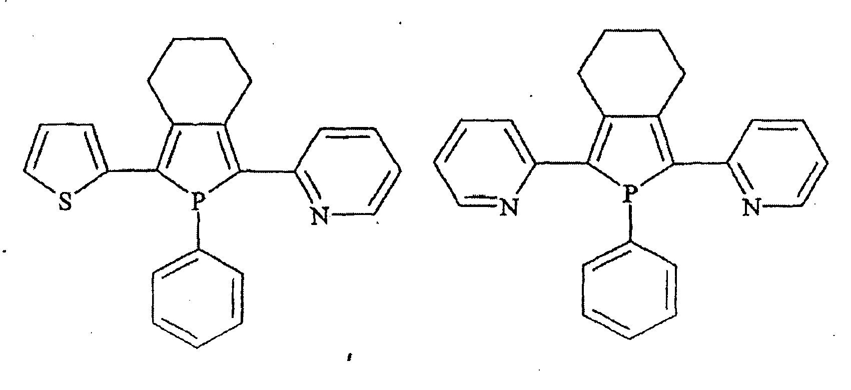

20. Medikament nach einem der Ansprüche 1 bis 19, **dadurch gekennzeichnet, dass** es als Wirkstoff mindestens eine Verbindung der allgemeinen Formel (A), und insbesondere der Formel (I) bis (VIII), in Kombination mit mindestens einer Krebs bekämpfenden Verbindung, wie etwa Cisplatin, Mitomycin C, Doxorubicin, Etoposid, Carmustin, umfasst.

21. Produkte, umfassend:
* mindestens eine Verbindung der allgemeinen Formel (A), und insbesondere der Formel (I) bis (VIII), wie in einem der Ansprüche 1 bis 19 definiert,
* und mindestens eine Krebs bekämpfende Verbindung, wie in Anspruch 20 definiert,
als Kombinationsprodukte für eine gleichzeitige, getrennte, oder über einen Zeitraum verteilte Verwendung in der Krebstherapie.

22. Kombinationsprodukt nach Anspruch 21, **dadurch gekennzeichnet, dass** es ein Produkt der Formel (A) und ein Krebs bekämpfendes Mittel in einem Verhältnis von etwa 0,1/1 bis etwa 2,5/1 und gegebenenfalls ein oder mehrere pharmazeutisch unbedenkliche Vehikel enthält.

23. Medikamente oder Kombinationsprodukte nach einem der Ansprüche 1 bis 22, **dadurch gekennzeichnet, dass** sie eine Form aufweisen, die intravenös verabreicht werden kann.

24. Medikamente oder Kombinationsprodukte nach einem der Ansprüche 1 bis 23, **dadurch gekennzeichnet, dass** die Dosierung der Verbindungen der Formel (A), die in diesen pharmazeutischen Zusammensetzungen oder Kombinationsprodukten enthalten ist, bei etwa 5 bis etwa 200 mg/m²/Tag oder pro Behandlung liegt.

25. Verwendung mindestens einer Verbindung der allgemeinen Formel (I), wie in einem der Ansprüche 2 bis 15 definiert, zur Herstellung eines Medikaments, das der Verbeugung oder der Behandlung von Pathologien dient, die mit einer übermäßigen Glutathionreductase-und/oder Thioredoxinreductaseaktivität zusammen hängen.

26. Verwendung nach Anspruch 25, **dadurch gekennzeichnet, dass** die Pathologien, die mit einer übermäßigen Glutathionreductase- und/oder Thioredoxinreductaseaktivität zusammen hängen, sind:
- Krebserkrankungen, und zwar alle festen oder flüssigen Tumore, und insbesondere Krebserkrankungen, die mit der Infektion mit dem Epstein-Barr-Virus assoziiert sind,
- rheumatoide Arthritis,
- Psoriasis oder Arthritis psoriatica,
- Sjögren-Syndrom,
- Infektion mit dem HIV-Virus und assoziierte Krankheiten, wie das Kaposi-Sarkom.

27. Verwendung mindestens einer Verbindung der Formel (VIII), wie in einem der Ansprüche 16 bis 19 definiert, zur Herstellung eines Medikaments, das der Behandlung von Krebserkrankungen dient.

28. Verwendung mindestens einer Verbindung der allgemeinen Formel (A), wie in einem der Ansprüche 1 bis 19 definiert, zur Herstellung eines Medikaments, das der Vorbeugung oder der Behandlung von Resistenzphänomenen gegenüber Krebs bekämpfenden Medikamenten, wie etwa Cisplatin, Mitomycin C, Doxorubicin, Etoposid, Carmustin und gegebenenfalls gegenüber infektionsbekämpfenden Medikamenten, wie etwa Metronidazol, Isoniazid dient.

29. Verbindung der folgenden allgemeinen Formel (IX): 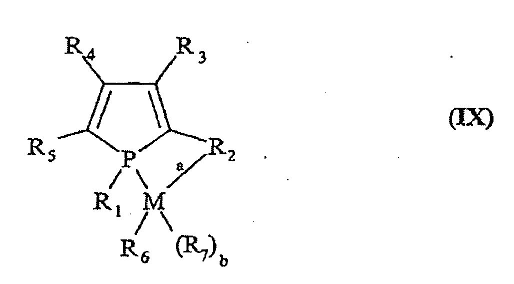 worin:
- a für eine Einfachbindung oder keine Bindung steht,
- b für 0 oder 1 steht,
- R₁ für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen, oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- R₂ für eine Stickstoff enthaltende Heteroarylgruppe oder einen Stickstoff enthaltenden Heteroring mit 5 oder 6 Atomen steht, der 1 bis 4 Stickstoffatome besitzt;
- R₃ und R₄ jeweils unabhängig voneinander für ein Wasserstoffatom oder eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen stehen,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen, wobei gegebenenfalls eine Alkylgruppe die Kohlenstoffatome an den Positionen 3 und 4 des Phospholrings untereinander verbinden kann, die jeweils die Substituenten R₃ und R₄ tragen, um einen Ring mit 3 bis 8 Kohlenstoffatomen zu bilden;
- R₅ für ein Wasserstoffatom, einen Heteroring mit fünf oder sechs Gliedern steht, der 1 bis 4 Stickstoff- oder Schwefelatome besitzt, oder für eine
• lineare oder verzweigte Alkylgruppe mit 1 bis 6 Kohlenstoffatomen,
• eine gesättigte oder ungesättigte Cycloalkylgruppe mit 3 bis 7 Kohlenstoffatomen,
• eine Arylgruppe mit 6 bis 14 Kohlenstoffatomen,
• eine lineare oder verzweigte Alkoxygruppe mit 1 bis 6 Kohlenstoffatomen,
• eine gesättigte oder ungesättigte Cycloalkoxygruppe mit 3 bis 7 Kohlenstoffatomen,
• eine Aryloxygruppe mit 6 bis 14 Kohlenstoffatomen,
• eine lineare oder verzweigte Alkylthiogruppe mit 1 bis 6 Kohlenstoffatomen,
• eine gesättigte oder ungesättigte Cycloalkylthiogruppe mit 3 bis 7 Kohlenstoffatomen oder
• eine Arylthiogruppe mit 6 bis 14 Kohlenstoffatomen steht,
die optional mit einer oder mehreren Gruppen, gleich oder verschieden, substituiert ist, ausgewählt aus einem Halogenatom, einer linearen oder verzweigten Alkylgruppe mit 1 bis 6 Kohlenstoffatomen, die optional mit einem oder mehreren Halogenatomen substituiert ist, oder einer Arylgruppe mit 6 bis 14 Kohlenstoffatomen;
- M für ein bivalentes, trivalentes oder tetravalentes Metallatom steht, unter der Bedingung, dass, wenn M für ein bivalentes Metallatom steht, a für keine Bindung steht und b gleich 0 ist, wenn M für ein trivalentes Metallatom steht, a für keine Bindung steht und b gleich 1 ist, und wenn M für eine tetravalentes Metallatom steht, a für eine Einfachbindung steht und b gleich 1 ist,
• R₆ für eine Thioglycosidgruppe mit 3 bis 60 Kohlenstoffatomen, die optional mit einer oder mehreren Gruppen, gleich oder verschieden, substituiert ist, ausgewählt aus den Schutzgruppen der Hydroxyl- oder Amingruppen, wie etwa einer Alkanoylgruppe mit 2 bis 6 Kohlenstoffatomen, oder einer Arylcarbonylgruppe mit 6 bis 14 Kohlenstoffatomen steht,
- R₇ für
• ein Halogenatom,
• eine Thioglycosidgruppe mit 3 bis 60 Kohlenstoffatomen, die optional mit einer oder mehreren Gruppen, gleich oder verschieden, substituiert ist, ausgewählt aus den Schutzgruppen der Hydroxyl- oder Amingruppen, wie etwa einer Alkanoylgruppe mit 2 bis 6 Kohlenstoffatomen, oder einer Arylcarbonylgruppe mit 6 bis 14 Kohlenstoffatomen,
• eine Peptidgruppe, die 1 bis 5 Aminosäuren umfasst, wobei mindestens eine der Aminosäuren eine Thiogruppe umfasst, wie etwa S-Cystein oder S-Glutathion, oder
• eine lineare oder verzweigte Thioalkylgruppe mit 1 bis 6 Kohlenstoffatomen, eine gesättigte oder ungesättigte Thiocycloalkylgruppe mit 3 bis 7 Kohlenstoffatomen oder eine Thioaryl- oder Thioheteroarylgruppe mit 6 bis 14 Kohlenstoffatomen, die optional mit einer oder mehreren Gruppen, gleich oder verschieden, substituiert ist, ausgewählt aus einem Halogenatom, einer Alkylgruppe mit 1 bis 6 Kohlenstoffatomen, die optional mit einem oder mehreren Halogenatomen substituiert ist, oder eine Arylgruppe mit 6 bis 14 Kohlenstoffatomen steht.

30. Verbindung nach Anspruch 29 der allgemeinen Formel (X): 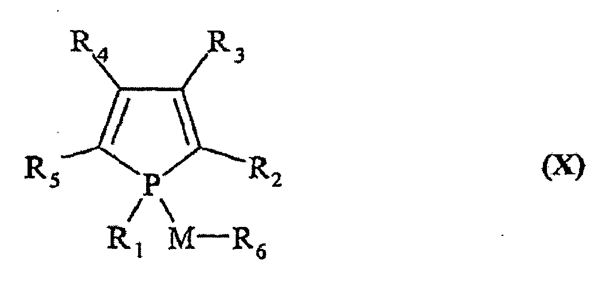 worin R₁, R₂, R₃, R₄, R₅ und R₆ sind, wie in Anspruch 29, und M für ein bivalentes Metallatom, wie etwa Au, steht.

31. Verbindung nach Anspruch 29 oder 30, der allgemeinen Formel (XI): 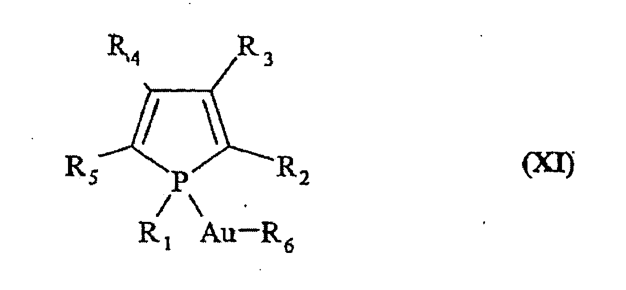
- worin R₂, R₃, R₄, R₅ und R₆ sind, wie in Anspruch 26 definiert,

32. Verbindung nach einem der Ansprüche 29 bis 31, worin:
- R₁ für eine Cycloalkylgruppe mit 3 bis 7 Kohlenstoffatomen oder eine Arylgruppe mit 6 bis 14 Kohlenstoffatomen, und insbesondere für eine Cyclohexyl- oder Phenylgruppe steht;
- R₂ für eine Stickstoff enthaltende Heteroarylgruppe mit sechs Atomen, die 1 bis 3 Stickstoffatome besitzt, und insbesondere für die 2-Pyridylgruppe steht;
- R₃ und R₄ unabhängig voneinander für Alkylgruppen mit 1 bis 6 Kohlenstoffatomen und insbesondere für eine Akylgruppe mit 3 oder 4 Kohlenstoffatomen stehen, die die Atome an den Positionen 3 und 4 des Phospholrings verbinden;
- R₅ für eine Arylgruppe mit 6 bis 14 Kohlenstoffatomen, eine substituierte oder nicht substituierte Thienyl- oder Pyridylgruppe, und insbesondere für eine Phenyl-, 2-Thienyl- oder 2-Pyridylgruppe steht;
- R₆ für ein Thiomonosacharid, vor allem ausgewählt aus Thiopentose oder Thiohexose, oder ein Thiodiosid, das optional mit einer oder mehreren Schutzgruppen der Hydroxyl- oder Amingruppen substituiert ist, und insbesondere Thioglucose, Thioglucosetetraacetat, Thiomannose, Thiomannosetetraacetat, Thiogalactose, Thiogalactosetetraacetat, Thioribose, Thioribosetriacetat, Thioxylose, Thioxylosetriacetat, Thioallose, Thioallosetetraacetat, Thiotalose, Thiotalosetetraacetat, Thiofucose, Thiofucosetetraacetat, Thio-N-acetyl-glucosamin, Thio-N-acetyl-glucosamintriacetat, Thio-N-acetylgalactosamin, Thio-N-acetylgalactosamintriacetat, Thio-N-acetylmannosamin, Thio-N-acetylmannosamintriacetat, Thiolactose, Thiolactoseheptaacetat steht.
- R₇ gegebenenfalls für ein Halogen, und insbesondere ein Chloratom, ein Thiomonosacharid, vor allem ausgewählt aus Thiopentose oder Thiohexose, oder ein Thiodiosid, das optional mit einer oder mehreren Schutzgruppen der Hydroxyl-oder Amingruppen substituiert ist, und insbesondere Thioglucose, Thioglucosetetraacetat, Thiomannose, Thiomannosetetraacetat, Thiogalactose, Thiogalactosetetraacetat, Thioribose, Thioribosetriacetat, Thioxylose, Thioxylosetriacetat, Thioallose, Thioallosetetraacetat, Thiotalose, Thiotalosetetraacetat, Thiofucose, Thiofucosetetraacetat, Thio-N-acetyl-glucosamin, Thio-N-acetyl-glucosamintriacetat, Thio-N-acetylgalactosamin, Thio-N-acetylgalactosamintriacetat, Thio-N-acetylmannosamin, Thio-N-acetylmannosamintriacetat, Thiolactose, Thiolactoseheptaacetat steht.

33. Verbindung nach einem der Ansprüche 29 bis 32 der folgenden Formel: 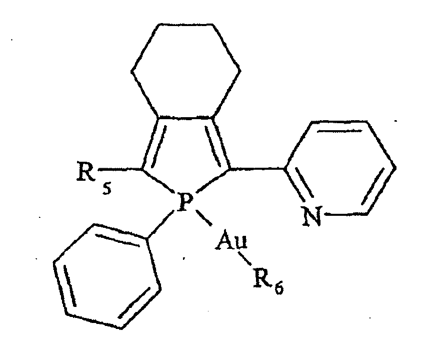 worin R₅ und R₆ sind, wie in Anspruch 32 definiert.

34. Verbindung nach einem der Ansprüche 29 bis 33 der folgenden Formel:
